(19) Europäisches Patentamt
European Patent Office
Office européen des brevets

(11) EP 4 001 308 A1

(12) EUROPEAN PATENT APPLICATION
published in accordance with Art. 153(4) EPC

(43) Date of publication:
**25.05.2022 Bulletin 2022/21**

(21) Application number: **20840792.4**

(22) Date of filing: **14.07.2020**

(51) International Patent Classification (IPC):
***C07K 16/28** (2006.01)* ***C12N 15/13** (2006.01)*
***A61K 39/395** (2006.01)* ***A61P 35/00** (2006.01)*

(52) Cooperative Patent Classification (CPC):
**A61K 39/395; A61P 35/00; C07K 16/28**

(86) International application number:
**PCT/CN2020/101883**

(87) International publication number:
**WO 2021/008523 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2019 CN 201910634309**

(71) Applicants:
- **Shanghai Junshi Biosciences Co., Ltd.**
  **Pilot Free Trade Zone**
  **Shanghai**
  **201210 (CN)**
- **Suzhou Junmeng Biosciences Co., Ltd.**
  **Jiangsu 215002 (CN)**

(72) Inventors:
- **MENG, Qin**
  **Suzhou, Jiangsu 215002 (CN)**
- **YAO, Jian**
  **Suzhou, Jiangsu 215002 (CN)**
- **FENG, Hui**
  **Suzhou, Jiangsu 215002 (CN)**
- **YAO, Sheng**
  **Suzhou, Jiangsu 215002 (CN)**
- **WU, Hai**
  **Suzhou, Jiangsu 215002 (CN)**

(74) Representative: **V.O.**
**P.O. Box 87930**
**2508 DH Den Haag (NL)**

# (54) ANTI-TIGIT ANTIBODIES AND APPLICATION THEREOF

(57) Provided are antibodies that bind specifically to TIGIT or antigen binding fragments of the antibodies and a composition thereof. Also provided are a nucleic acid molecule coding the antibodies or the antigen binding fragments thereof, an expression vector and a host cell for expressing the antibodies or the antigen binding fragments thereof, and therapeutic and diagnostic uses of the antibodies.

EP 4 001 308 A1

**Description**

## TECHNICAL FIELD

The present invention relates to anti-TIGIT antibodies and use of these antibodies and compositions thereof in the treatment of cancers.

## BACKGROUND

TIGIT (T cell immunoreceptor with Ig and ITIM domains) is an immunoregulatory receptor consisting of an extracellular immunoglobulin domain, a type I transmembrane region and two ITIM motifs and expressed predominantly on activated T cells and NK cells (Stanietsky et al., PNAS.2009, 106, 17858-17863). TIGIT recognizes ligands poliovirus receptor (PVR, CD155) and the Nectin 2 (PVRL2/CD112), which are overexpressed on a variety of different tumor cells. It has been reported that TIGIT binds to the ligand PVR with high affinity. The binding of TIGIT to the ligand will activate inhibitory signals mediated by the following two motifs present in the cytoplasmic tail of TIGIT: an immunoreceptor tail tyrosine (ITT) -like motif and an immunodominant tyrosine-based inhibitory (ITIM) motif (Liu et al., Cell death and differentiation 2013, 20, 456-464; Stanietsky et al., European journal of immunology, 2013, 43, 2138-2150). The tumor cell surface ligand, through its binding to the ITIM domain of TIGIT on the surfaces of NK cells and T cells, inhibits the cytotoxicity of the NK cells and the activity of the T cells, and thereby mediates the immune evasion mechanism of tumor cells. It has been reported in a number of patent documents that anti-TIGIT antibodies which block the binding of TIGIT to its ligand can be used to treat diseases such as tumors by inhibiting TIGIT-mediated immunosuppression (WO2004/024068, WO2009/126688, WO2015/009856, and WO2016/028656).

There is an unmet need to provide other more effective, specific, safe and/or stable pharmaceutical agents that, alone or in combination with other pharmaceutical agents, can enable cells of the immune system to attack tumor cells by decreasing the inhibitory activity of human TIGIT.

## BRIEF SUMMARY

The present invention provides an anti-TIGIT antibody or an antigen-binding fragment thereof characterized by having a unique CDR sequence composition and being capable of binding to TIGIT with high affinity and high specificity. The anti-TIGIT antibody or the antigen-binding fragment thereof provided herein can be used as an independent therapy or in combination with other therapies and/or other anti-cancer pharmaceutical agents to treat, for example, cancers.

One aspect of the present invention provides an antibody or an antigen-binding fragment thereof binding to TIGIT with high specificity.

In some embodiments, the anti-TIGIT antibody or the antigen-binding fragment thereof described herein comprises 1 to 3 heavy chain complementarity determining regions selected from heavy chain complementarity determining regions HCDR1, HCDR2 and HCDR3, wherein the HCDR1 comprises an amino acid sequence identical to or having at least 90%, 91%, 92%, 93%, 94%, 95%, 96%, 97%, 98% or 99% identity to an amino acid sequence selected from SEQ ID NOs: 1 and 11, the HCDR2 comprises an amino acid sequence identical to or having at least 90%, 91%, 92%, 93%, 94%, 95%, 96%, 97%, 98% or 99% identity to an amino acid sequence selected from SEQ ID NOs: 2 and 12, and the HCDR3 comprises an amino acid sequence identical to or having at least 90%, 91%, 92%, 93%, 94%, 95%, 96%, 97%, 98% or 99% identity to an amino acid sequence selected from SEQ ID NOs: 3 and 13.

In some embodiments, the anti-TIGIT antibody or the antigen-binding fragment thereof described herein comprises heavy chain complementarity determining regions HCDR1, HCDR2 and HCDR3, wherein the HCDR1 comprises an amino acid sequence set forth in SEQ ID NO: 1, the HCDR2 comprises an amino acid sequence set forth in SEQ ID NO: 2, and the HCDR3 comprises an amino acid sequence set forth in SEQ ID NO: 3.

In some embodiments, the anti-TIGIT antibody or the antigen-binding fragment thereof described herein comprises heavy chain complementarity determining regions HCDR1, HCDR2 and HCDR3, wherein the HCDR1 comprises an amino acid sequence set forth in SEQ ID NO: 11, the HCDR2 comprises an amino acid sequence set forth in SEQ ID NO: 12, and the HCDR3 comprises an amino acid sequence set forth in SEQ ID NO: 13.

In some embodiments, the anti-TIGIT antibody or the antigen-binding fragment thereof described herein comprises 1 to 3 light chain complementarity determining regions selected from light chain complementarity determining regions LCDR1, LCDR2 and LCDR3, wherein the LCDR1 comprises an amino acid sequence identical to or having at least 90%, 91%, 92%, 93%, 94%, 95%, 96%, 97%, 98% or 99% identity to an amino acid sequence set forth in SEQ ID NO: 6 or 16, the LCDR2 comprises an amino acid sequence identical to or having at least 90%, 91%, 92%, 93%, 94%, 95%, 96%, 97%, 98% or 99% identity to an amino acid sequence set forth in SEQ ID NO: 7 or 17, and the LCDR3 comprises an amino acid sequence identical to or having at least 90%, 91%, 92%, 93%, 94%, 95%, 96%, 97%, 98% or 99% identity to an amino acid sequence set forth in SEQ ID NO: 8 or 18.

In some embodiments, the anti-TIGIT antibody or the antigen-binding fragment thereof described herein comprises light chain complementarity determining regions LCDR1, LCDR2 and LCDR3, wherein the LCDR1 comprises an amino acid sequence set forth in SEQ ID NO: 6, the LCDR2 comprises an amino acid sequence set forth in SEQ ID NO: 7, and the LCDR3 comprises an amino acid sequence set forth in SEQ ID NO: 8.

In some embodiments, the anti-TIGIT antibody or the antigen-binding fragment thereof described herein comprises light chain complementarity determining regions LCDR1, LCDR2 and LCDR3, wherein the LCDR1 comprises an amino acid sequence set forth in SEQ ID NO: 16, the LCDR2 comprises an amino acid sequence set forth in SEQ ID NO: 17, and the LCDR3 comprises an amino acid sequence set forth in SEQ ID NO: 18.

In some embodiments, the antibody or the antigen-binding fragment thereof described herein comprises heavy chain complementarity determining regions HCDR1, HCDR2 and HCDR3 and light chain complementarity determining regions LCDR1, LCDR2 and LCDR3, wherein the HCDR1 comprises an amino acid sequence set forth in SEQ ID NO: 1, the HCDR2 comprises an amino acid sequence set forth in SEQ ID NO: 2, the HCDR3 comprises an amino acid sequence set forth in SEQ ID NO: 3, the LCDR1 comprises an amino acid sequence set forth in SEQ ID NO: 6, the LCDR2 comprises an amino acid sequence set forth in SEQ ID NO: 7, and the LCDR3 comprises an amino acid sequence set forth in SEQ ID NO: 8.

In some embodiments, the antibody or the antigen-binding fragment thereof described herein comprises heavy chain complementarity determining regions HCDR1, HCDR2 and HCDR3 and light chain complementarity determining regions LCDR1, LCDR2 and LCDR3, wherein the HCDR1 comprises an amino acid sequence set forth in SEQ ID NO: 11, the HCDR2 comprises an amino acid sequence set forth in SEQ ID NO: 12, the HCDR3 comprises an amino acid sequence set forth in SEQ ID NO: 13, the LCDR1 comprises an amino acid sequence set forth in SEQ ID NO: 16, the LCDR2 comprises an amino acid sequence set forth in SEQ ID NO: 17, and the LCDR3 comprises an amino acid sequence set forth in SEQ ID NO: 18.

In some embodiments, the anti-TIGIT antibody or the antigen-binding fragment thereof described herein comprises a heavy chain variable region (VH), wherein the heavy chain variable region (VH) comprises an amino acid sequence identical to or having at least 90%, 91%, 92%, 93%, 94%, 95%, 96%, 97%, 98% or 99% identity to any amino acid sequence selected from SEQ ID NOs: 4, 14, 21, 23, 25, 27 and 29.

In some embodiments, the anti-TIGIT antibody or the antigen-binding fragment thereof described herein comprises a light chain variable region (VL), wherein the light chain variable region (VL) comprises an amino acid sequence identical to or having at least 90%, 91%, 92%, 93%, 94%, 95%, 96%, 97%, 98% or 99% identity to any amino acid sequence selected from SEQ ID NOs: 9, 19, 22, 24, 26, 28 and 30.

In some embodiments, the anti-TIGIT antibody or the antigen-binding fragment thereof described herein comprises a heavy chain variable region (VH) and a light chain variable region (VL), wherein the VH comprises an amino acid sequence identical to or having at least 90%, 91%, 92%, 93%, 94%, 95%, 96%, 97%, 98% or 99% identity to any amino acid sequence selected from SEQ ID NOs: 4, 14, 21, 23, 25, 27, and 29; and wherein VL comprises an amino acid sequence identical to or having at least 90%, 91%, 92%, 93%, 94%, 95%, 96%, 97%, 98% or 99% identity to any amino acid sequence selected from SEQ ID NOs: 9, 19, 22, 24, 26, 28 and 30.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 4, and the VL comprises an amino acid sequence set forth in SEQ ID NO: 9.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 14, and the VL comprises an amino acid sequence set forth in SEQ ID NO: 19.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 21, and the VL comprises an amino acid sequence set forth in SEQ ID NO: 22.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 23, and the VL comprises an amino acid sequence set forth in SEQ ID NO: 24.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 25, and the VL comprises an amino acid sequence set forth in SEQ ID NO: 26.

In some embodiments, the anti-TIGIT antibody or the antigen-binding fragment thereof described herein comprises a heavy chain variable regions (VH) and a light chain variable region (VL), wherein the VH comprises an amino acid sequence set forth in SEQ ID NO: 27, and the VL comprises an amino acid sequence set forth in SEQ ID NO: 28.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 29, and the VL comprises an amino acid sequence set forth in SEQ ID NO: 30.

In some embodiments, the antibody or the antigen-binding fragment thereof described herein comprises a light chain and/or a heavy chain, wherein an amino acid sequence of the heavy chain is set forth in SEQ ID NO: 31 or 32, and/or an amino acid sequence of the light chain is set forth in SEQ ID NO: 32 or 34.

In some embodiments, the amino acid sequence of the heavy chain of the antibody described herein is set forth in SEQ ID NO: 31, and the amino acid sequence of the light chain is set forth in SEQ ID NO: 32; or the amino acid sequence of the heavy chain of the antibody is set forth in SEQ ID NO: 33, and the amino acid sequence of the light chain is set forth in SEQ ID NO: 34.

In some embodiments, the antibody or the antigen-binding fragment thereof described herein has the property of cross-binding to human and cynomolgus monkey TIGIT proteins.

In some embodiments, the antibody or the antigen-binding fragment thereof described herein has the property of blocking or inhibiting the binding of TIGIT to its natural ligands PVR or/and PVRL2.

In some embodiments, the antibody or the antigen-binding fragment thereof described herein blocks or inhibits TIGIT-mediated bioactivity.

In some embodiments, the anti-TIGIT antibody or the antigen-binding fragment thereof described herein is a murine antibody, a chimeric antibody, a humanized antibody or a fully human antibody.

In some embodiments, the anti-TIGIT antibody or the antigen-binding fragment thereof described herein is a complete antibody, a single-chain antibody, a Fab antibody, a Fab' antibody, a $(Fab')_2$ antibody or a bispecific (multispecific) antibody.

In some embodiments, the anti-TIGIT antibody or the antigen-binding fragment thereof described herein is of any IgG subtype, such as IgG1, IgG2, IgG3 or IgG4.

The present invention provides an isolated nucleic acid molecule, which encodes the aforementioned antibody or antigen-binding fragment thereof.

The present invention provides a recombinant or expression vector, which comprises one or more aforementioned nucleic acid sequences, wherein the vector is suitable for recombinant production of the aforementioned antibody or antigen-binding fragment thereof.

The present invention provides a host cell, which comprises one or more aforementioned recombinant or expression vectors or aforementioned nucleic acid molecules, or expresses the antibody of any of the aforementioned embodiments.

The present invention provides a pharmaceutical composition, which comprises a composition of the aforementioned antibody or antigen-binding fragment thereof and a pharmaceutically acceptable carrier or excipient.

In some embodiments, the aforementioned pharmaceutical composition further comprises a PD-1 axis antagonist.

In some embodiments, the anti-TIGIT antibody or the antigen-binding fragment thereof described herein has at least one of the following properties:

1) binding to human TIGIT with a $K_D$ of at least about 5 nM, at least about 1 nM, at least about 0.1 nM, at least about 0.01 nM or at least about 0.001 nM;
2) cross-reacting with cynomolgus monkey TIGIT.

A second aspect of the present invention also relates to a method for producing the antibody or the antigen-binding fragment thereof described herein. Such a method comprises providing an isolated nucleic acid molecule encoding the antibody or the antigen-binding fragment thereof described herein or an expression vector comprising such a nucleic acid molecule, particularly a vector for recombinant production of the antibody or the antigen-binding fragment thereof described herein in a host cell.

A third aspect of the present invention also relates to a host cell comprising one or more aforementioned recombinant or expression vectors and a method for producing the antibody or the antigen-binding fragment thereof described herein, wherein the method comprises: culturing the host cell and purifying and isolating the antibody or the antigen-binding fragment thereof.

The present invention also provides a method for producing the aforementioned antibody or the antigen-binding fragment thereof, which comprises: culturing the aforementioned host cell, and isolating the antibody or the antigen-binding fragment thereof from the culture.

A fourth aspect of the present invention also relates to a method for treating cancer in a subject, which comprises: administering to the subject an effective amount of any of the anti-TIGIT antibodies or the antigen-binding fragments thereof or the pharmaceutical compositions thereof described herein.

In one embodiment, the present invention also relates to use of any of the anti-TIGIT antibodies or the antigen-binding fragments thereof described herein in preparing a medicament for treating cancer in a subject.

The present invention also provides use of the aforementioned antibody or antigen-binding fragment thereof, nucleic acid molecule, vector, host cell or pharmaceutical composition in preparing a medicament for treating and/or preventing a TIGIT-mediated disease or disorder, wherein preferably, the disease or disorder is cancer.

A fifth aspect of the present invention also relates to co-administration of one or more therapies (e.g., treatment modalities and/or other therapeutic agents) to a subject with cancer. In some embodiments, the aforementioned therapy comprises: administering to the subject an effective amount of the anti-TIGIT antibody or the antigen-binding fragment thereof or the pharmaceutical composition thereof according to any of the embodiments described herein. In some embodiments, the treatment modalities include surgical treatment and radiation therapy. In some embodiments, the aforementioned other therapeutic agents are selected from chemotherapeutic agents, PD-1 axis antagonists and other tumor-targeting therapeutic agents, wherein the PD-1 axis antagonists are preferred.

In some embodiments, the present invention also relates to use of a combination of any of the anti-TIGIT antibodies or the antigen-binding fragments thereof described herein and a PD-1 axis antagonist in preparing a medicament for treating cancer in a subject.

In some embodiments, the PD-1 axis antagonist is selected from an anti-PD-1 antibody, an anti-PD-Ll antibody and an anti-PD-L2 antibody.

A sixth aspect of the present invention also relates to a method for detecting TIGIT in a sample, which comprises: a) contacting the sample with any of the anti-TIGIT antibodies or the fragments thereof described herein; and b) detecting formation of a complex of the anti-TIGIT antibody or the fragment thereof with TIGIT. In one embodiment, the anti-TIGIT antibody is detectably labeled.

In some embodiments, the present invention relates to a kit or an article of manufacture, which comprises any of the anti-TIGIT antibodies or the fragments thereof described herein. In some embodiments, the kit or the article of manufacture comprises the anti-TIGIT antibody or the fragment thereof described herein, optionally a pharmaceutically acceptable excipient, and optionally one or more other therapeutic agents (e.g., a chemotherapeutic agent, a PD-1 axis antagonist or a tumor-targeting therapeutic agent).

The present invention also encompasses any combination of the embodiments described herein. Any of the embodiments described herein or any combination thereof is applicable to any and all of the anti-TIGIT antibodies or the fragments thereof, the methods, and the uses described herein.

## BRIEF DESCRIPTION OF THE DRAWINGS

FIG. 1 illustrates the effect of hybridoma antibodies on the activity of T cells detected by luciferase assay.
FIG. 2 illustrates the binding of chimeric antibodies to TIGIT detected by Elisa.
FIG. 3 illustrates the effect of the chimeric antibodies on the activity of T cells detected by luciferase assay.
FIG. 4 illustrates the binding of humanized antibodies to TIGIT detected by ELISA.
FIG. 5 illustrates the effect of the humanized antibodies on the activity of T cells detected by luciferase assay.
FIG. 6 illustrates the effect of the humanized antibodies in blocking the binding of TIGIT to the ligand PVR detected by FACS.
FIG. 7 illustrates a study on the efficacy of the humanized TIGIT antibodies and their combination with an anti-PD-1 antibody in MC38 tumor-bearing TIGIT transgenic mice.

## DETAILED DESCRIPTION

The present invention provides an anti-TIGIT antibody or an antigen-binding fragment thereof characterized by having a unique CDR sequence composition and being capable of binding to TIGIT with high affinity and high specificity. The anti-TIGIT antibody or the antigen-binding fragment thereof provided herein can be used as an independent therapy or in combination with other therapies and/or other anti-cancer pharmaceutical agents to treat, for example, cancers.

### Definitions

Unless otherwise stated, embodiments of the present invention will employ conventional techniques of molecular biology (including recombinant techniques), microbiology, cytobiology, biochemistry and immunology, which are all within the skill of the art.

In order to facilitate the understanding of the present invention, some technical and scientific terms are specifically defined as follows. Unless otherwise specifically defined herein, all technical and scientific terms used herein have the same meaning as commonly understood by those of ordinary skill in the art to which the present invention belongs. For definitions and terminology in the art, the skilled person can refer specifically to Current Protocolsin Molecular Biology (Ausubel). Abbreviations for amino acid residues are standard 3-letter and/or 1-letter codes used in the art to denote one of the 20 commonly used L-amino acids. The singular forms used herein (including claims) include their plural forms, unless otherwise specified in the context explicitly.

The term "about" used in combination with a numerical value is intended to encompass the numerical values in a range from a lower limit less than the specified numerical value by 5% to an upper limit greater than the specified numerical value by 5%.

As used herein, the term "TIGIT", the full name of which is T cell immunoreceptor with Ig and ITIM domains, refers to any natural TIGIT from any vertebrate animal (including mammals, such as primates (e.g., human) and rodents (e.g., mice and rats)), unless otherwise indicated. This term encompasses "full-length" unprocessed TIGIT and TIGIT in any form resulting from intracellular processing or any fragment thereof. The term also includes variants of naturally occurring TIGIT, e.g., splicing variants or allelic variants. In one embodiment, TIGIT refers to full-length TIGIT from human and cynomolgus monkey or fragments thereof (such as mature fragments thereof lacking a signal peptide). In one embodiment, human TIGIT refers to a mature TIGIT identical to a sequence of amino acid residues 22-244 (Genbank accession No. NP_776160.2) (SEQ ID NO: 31) (amino acid residues 1-21 are leader peptides). In one embodiment, human TIGIT refers to a TIGIT extracellular domain identical to a sequence of amino acid residues 22-141 (Genbank accession No. NP_776160.2). In one embodiment, the cynomolgus monkey (Wacaca fascicularis) TIGIT refers to a mature TIGIT identical to a sequence of amino acid residues 22-245 (Genbank accession No. XP_005548158.1).

The "percent (%) amino acid sequence identity" is defined as the percentage of amino acid residues in a candidate sequence that are identical to those in a reference polypeptide sequence after aligning the sequences (with gaps introduced if necessary) to achieve maximum percent sequence identity without considering any conservative substitution as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring alignment, including any algorithm required to obtain maximum alignment for the full length of the aligned sequences.

The term "immune response" refers to the action of, for example, lymphocytes, antigen-presenting cells, phagocytes, granulocytes, and soluble macromolecules produced by the above cells or liver (including antibodies, cytokines and complements) that results in selective damage to, destruction of, or elimination from the human body of invading pathogens, cells or tissues infected with pathogens, cancerous cells, or, in the cases of autoimmunity or pathological inflammation, normal human cells or tissues.

The term "signal transduction pathway" or "signal transduction activity" refers to a biochemical causal relationship generally initiated by a protein-protein interaction (such as binding of a growth factor to a receptor) and resulting in transmission of a signal from one portion of a cell to another portion of the cell. In general, the transmission includes specific phosphorylation of one or more tyrosine, serine or threonine residues on one or more proteins in a series of reactions causing signal transduction. The penultimate process typically involves a nuclear event, resulting in a change in gene expression.

The term "activity" or "bioactivity", or the term "biological property" or "biological characteristic" can be used interchangeably herein and includes, but is not limited to, epitope/antigen affinity and specificity, the ability to neutralize or antagonize TIGIT activity *in vivo* or *in vitro,* $IC_{50}$, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain high expression level of the protein in mammalian cells. The aforementioned properties or characteristics are observed, determined or assessed using techniques well known in the art, including but not limited to ELISA, FACS or BIACORE plasma resonance analysis, unlimited *in vitro* or *in vivo* neutralization assays, receptor binding, cytokine or growth factor production and/or secretion, signal transduction, and immunohistochemistry of tissue sections of different origins (including human, primate or any other origin).

An "antibody" refers to any form of antibody having a desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

An "isolated antibody" refers to the purified state of a binding compound, and, in this case, means that the molecule is substantially free of other biomolecules, such as nucleic acids, proteins, lipids, sugars, or other substances such as cell debris and growth medium. The term "isolate(d)" does not mean the complete absence of such substances or the absence of water, buffers or salts, unless they are present in amounts that will significantly interfere with the experimental or therapeutic use of the binding compounds described herein.

A "monoclonal antibody" refers to an antibody obtained from a substantially homogeneous population of antibodies, i.e., the antibodies comprising the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

A "full-length antibody" refers to an immunoglobulin molecule comprising four peptide chains when present naturally, including two heavy (H) chains (about 50-70 kDa in full length) and two light (L) chains (about 25 kDa in full length) linked to each other by disulfide bonds. Each heavy chain consists of a heavy chain variable region (abbreviated herein as VH) and a heavy chain constant region (abbreviated herein as CH). The heavy chain constant region consists of 3 domains CHI, CH2 and CH3. Each light chain consists of a light chain variable region (abbreviated herein as VL) and a light chain constant region. The light chain constant region consists of one domain CL. The VH and VL regions can be further divided into complementarity determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains contain binding domains that interact with antigens. The constant regions of an antibody can mediate the binding of immunoglobulins to host tissues or factors, including the binding of various cells of the immune system (e.g., effector cells) to the first component (Clq) of a classical complement system.

An "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of binding fragments of an antibody include, but are not limited to, Fab, Fab', $F(ab')_2$ and Fv fragments; a diabody; a linear antibody; a single-chain antibody molecule, such as sc-Fv; and a nanobody and a multispecific antibody formed by fragments of the antibody. A binding fragment or a derivative generally retains at least 10% of its antigen-binding activity when the antigen-binding activity is expressed on a molar concentration basis. Preferably, the binding fragment or derivative retains at least 20%, 50%, 70%, 80%, 90%, 95% or 100% or more of the antigen-binding affinity of the parent antibody. It is also contemplated that an antigen-binding fragment of an antibody may include conservative or non-conservative amino acid substitutions that do not significantly alter its bioactivity (referred to as "conservative variants" or "function-conservative variants" of the antibody). The term "binding compound" refers to both an antibody and a binding fragment thereof.

A "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen-binding.

A "domain antibody" is an immunofunctional immunoglobulin fragment that contains only the heavy chain variable region or the light chain variable region. In certain cases, two or more VH regions are covalently linked to a peptide linker to form a bivalent domain antibody. The two VH regions of the bivalent domain antibody may target the same or different antigens.

A "bivalent antibody" comprises two antigen-binding sites. In certain cases, the two binding sites have the same antigen specificity. However, a bivalent antibody may be bispecific.

A "diabody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of the other chain to form two antigen-binding sites.

A "chimeric antibody" is an antibody having the variable domains of a first antibody and the constant domains of a second antibody, wherein the first and second antibodies are from different species. Typically, the variable domain is obtained from an antibody of an experimental animal such as a rodent ("parent antibody"), and the constant domain sequence is obtained from a human antibody, such that the resulting chimeric antibody is less likely to induce an adverse immune response in a human subject as compared to the parent rodent antibody.

A "humanized antibody" refers to an antibody form containing sequences from both human and non-human (such as mouse and rat) antibodies. In general, a humanized antibody comprises substantially all of at least one, and typically two, variable domains, in which all or substantially all of the hypervariable loops correspond to those of a non-human immunoglobulin and all or substantially all of the framework regions (FRs) are those of a human immunoglobulin sequence. The humanized antibody may optionally comprise at least a portion of a human immunoglobulin constant region (Fc).

A "fully human antibody" refers to an antibody that comprises only human immunoglobulin sequences. A fully human antibody may contain mouse glycochains if produced in mice, mouse cells or hybridomas derived from mouse cells. Likewise, a "mouse antibody" refers to an antibody that comprises only mouse immunoglobulin sequences. Alternatively, a fully human antibody may contain rat glycochains if produced in rats, rat cells or hybridomas derived from rat cells. Likewise, a "rat antibody" refers to an antibody that comprises only rat immunoglobulin sequences.

"Isotypes" of antibodies refer to types of antibodies (e.g., IgM, IgE and IgG (such as IgG1, IgG2 or IgG4)) provided by heavy chain constant region genes. Isotype also includes modified forms of one of these types in which modifications have been made to alter Fc function, for example to enhance or attenuate effector function or binding to Fc receptors.

The term "PD-1 axis antagonist" refers to a molecule which inhibits the interaction of a PD-1 axis binding ligand with one or more of its binding ligands, thereby removing T cell dysfunction resulting from signaling on a PD-1 signaling axis, a result of which is the restoration or enhancement of T cell function (e.g., proliferation, cytokine production and target-cell killing). As used herein, PD-1 axis antagonists include PD-1 antagonists (e.g., anti-PD-1 antibodies), PD-L1 antagonists (e.g., anti-PD-Ll antibodies), and PD-L2 antagonists (e.g., anti-PD-L2 antibodies).

The term "PD-1 antagonist" refers to a molecule which reduces, blocks, inhibits, eliminates or interferes with signal transduction resulting from the interaction of PD-1 with one or more of its binding ligands (such as PD-L1 and PD-L2). In some embodiments, the PD-1 antagonist is a molecule which inhibits the binding of PD-1 to one or more of its binding ligands. In some specific embodiments, the PD-1 antagonist inhibits the binding of PD-1 to PD-L1 and/or PD-L2. For example, the PD-1 antagonist includes an anti-PD-1 antibody, an antigen-binding fragment thereof, an immunoadhesin, a fusion protein, an oligopeptide and other molecules that reduce, block, inhibit, eliminate or interfere with signal transduction resulting from the interaction of PD-1 with PD-L1 and/or PD-L2. In one embodiment, the PD-1 antagonist reduces negative co-stimulatory signals mediated by or via cell surface proteins expressed on T lymphocytes (signal transduction mediated via PD-1), thereby rendering dysfunctional T cells less dysfunctional (e.g., enhancing effector response to antigen recognition). In some embodiments, the PD-1 antagonist is an anti-PD-1 antibody. In a specific embodiment, the PD-1 antagonist is nivolumab, pembrolizumab, pidilizumab, or any anti-PD-1 antibody or antigen-binding fragment thereof disclosed in WO2014/206107.

The term "PD-L1 antagonist" refers to a molecule which reduces, blocks, inhibits, eliminates or interferes with signal transduction resulting from the interaction of PD-L1 with one or more of its binding ligands (such as PD-1 and B7-1). In some embodiments, it is a molecule which inhibits the binding of PD-L1 to its binding ligands. In a particular aspect, the PD-L1 antagonist inhibits the binding of PD-L1 to PD-1 and/or B7-1. In some embodiments, the PD-L1 antagonist includes an anti-PD-Ll antibody, an antigen-binding fragment thereof, an immunoadhesin, a fusion protein, an oligopeptide and other molecules that reduce, block, inhibit, eliminate or interfere with signal transduction resulting from the interaction of PD-L1 with one or more of its binding ligands (such as PD-1 and B7-1). In one embodiment, the PD-L1 antagonist reduces negative co-stimulatory signals mediated by or via cell surface proteins expressed on T lymphocytes (signal transduction mediated via PD-L1), thereby enhancing the activity of T cells (e.g., enhancing effector response to antigen recognition). In some embodiments, the PD-L1 antagonist is an anti-PD-Ll antibody. In a specific aspect, the anti-PD-Ll antibody is atezolizumab, durvalumab or any anti-PD-Ll antibody or an antigen-binding fragment thereof disclosed in WO2018/153320.

The term "PD-L2 antagonist" refers to a molecule which reduces, blocks, inhibits, eliminates or interferes with signal transduction resulting from the interaction of PD-L2 with one or more of its binding ligands (such as PD-1). In some embodiments, the PD-L2 antagonist is a molecule which inhibits the binding of PD-L2 to one or more of its binding ligands. In a particular aspect, the PD-L2 antagonist inhibits the binding of PD-L2 to PD-1. In some embodiments, the PD-L2 antagonist includes an anti-PD-L2 antibody, an antigen-binding fragment thereof, an immunoadhesin, a fusion protein, an oligopeptide and other molecules that reduce, block, inhibit, eliminate or interfere with signal transduction resulting from the interaction of PD-L2 with one or more of its binding ligands (such as PD-1). In one embodiment, the PD-L2 binding antagonist reduces negative co-stimulatory signals mediated by or via cell surface proteins expressed on T lymphocytes (signal transduction mediated via PD-L2), thereby enhancing the activity of T cells (e.g., enhancing effector response to antigen recognition).

The term "etigilimab" refers to an antibody identical to a sequence with CAS Registry Number: 2044984-83-8, or an antibody of its isotype. In a specific embodiment, the etigilimab is of an IgG4 isotype.

The term "nucleic acid" or "polynucleotide" refers to deoxyribonucleic acid (DNA) or ribonucleic acid (RNA) and polymers thereof in either single- or double-stranded form. Unless explicitly limited, the term includes nucleic acids containing known analogs of natural nucleotides that have binding properties similar to that of the reference nucleic acid and are metabolized in a manner similar to naturally occurring nucleotides (see U.S. Pat. No. 8,278,036 to Kariko et al., which discloses an mRNA molecule with uridine replaced by pseudouridine, a method for synthesizing the mRNA molecule, and a method for delivering a therapeutic protein *in vivo*). Unless otherwise specified, a particular nucleic acid sequence also implicitly includes conservatively modified variants thereof (e.g., degenerate codon substitutions), alleles, orthologs, SNPs, and complementary sequences as well as the sequence explicitly indicated. Specifically, degenerate codon substitutions can be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

A "construct" refers to any recombinant polynucleotide molecule (such as plasmid, cosmid, virus, autonomously replicating polynucleotide molecule, phage, or linear or circular single- or double-stranded DNA or RNA polynucleotide molecule) derived from any source, capable of genomic integration or autonomous replication, and comprising a polynucleotide molecule where one or more polynucleotide molecules have been linked in a functionally operative manner (i.e., operably linked). The recombinant construct typically comprises a polynucleotide of the present invention operably linked to transcription initiation regulatory sequences that will direct transcription of the polynucleotide in a host cell. Both heterologous and non-heterologous (i.e., endogenous) promoters can be used to direct expression of the nucleic acids of the present invention.

A "vector" refers to any recombinant polynucleotide construct that can be used for transformation purpose (i.e., the introduction of heterologous DNA into a host cell). One type of vector is a "plasmid", which refers to a double-stranded DNA loop into which additional DNA segments can be ligated. Another type of vector is a viral vector, in which additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into genome of the host cell upon introduction into a host cell, and are thereby replicated along with the host genome. In addition, certain vectors are capable of directing the expression of operably linked genes. Such vectors are referred to herein as "expression vectors".

The term "expression vector" as used herein refers to a nucleic acid molecule capable of replicating and expressing a target gene when transformed, transfected or transduced into a host cell. The expression vector comprises one or more phenotypic selectable markers and an origin of replication to ensure maintenance of the vector and to provide amplification in the host if needed.

Unless otherwise or explicitly specified in the context, "activation", "stimulation" and "treatment" for a cell or a receptor may have the same meaning. For example, the cell or the receptor is activated, stimulated or treated with a ligand. "Ligands" include natural and synthetic ligands, such as cytokines, cytokine variants, analogs, mutant proteins, and binding compounds derived from antibodies. "Ligands" also include small molecules, such as peptidomimetics of cytokines and peptidomimetics of antibodies. "Activation" may refer to the activation of a cell regulated by internal mechanisms and external or environmental factors. "Response/reaction", e.g., a response of a cell, a tissue, an organ or an organism, includes changes in biochemical or physiological behaviors (e.g., concentration, density, adhesion or migration, gene expression rate, or differentiation state within a biological compartment), where the changes are associated with activation, stimulation or treatment, or are associated with an internal mechanism such as genetic programming.

As used herein, the term "treat", "treating" or "treatment" of any disease or disorder refers in one embodiment to ameliorating the disease or disorder (i.e., slowing or arresting or reducing the progression of the disease or at least one of its clinical symptoms). In another embodiment, "treat", "treating" or "treatment" refers to ameliorating or alleviating at least one physical parameter, including those physical parameters that may not be discernible by the patient. In another embodiment, "treat", "treating" or "treatment" refers to modulating the disease or disorder, physically (e.g., stabilization of discernible symptoms), physiologically (e.g., stabilization of physical parameters), or both. Unless explicitly described herein, methods for assessing treatment and/or prevention of a disease are generally known in the art.

A "subject" includes any human or non-human animal. The term "non-human animal" includes all vertebrates, e.g., mammals and non-mammals, such as non-human primates, sheep, dog, cat, horse, cattle, chicken, amphibians, and reptiles. As used herein, the term "cyno" refers to a cynomolgus monkey.

Administration "in combination with" one or more other therapeutic agents includes simultaneous (co-) administration and sequential administration in any order.

"Therapeutically effective amount", "therapeutically effective dose" and "effective amount" refer to an amount of the anti-TIGIT antibody or the antigen-binding fragment thereof disclosed herein that is effective in preventing or ameliorating one or more symptoms of a disease or condition or the progression of the disease or condition when administered alone or in combination with other therapeutic drugs to a cell, a tissue or a subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will result in an increase in a diagnostic index or parameter by at least 10%, generally at least 20%, preferably at least about 30%, more preferably at least 40%, and most preferably at least 50%.

"Cancer" and "cancerous" refer to or describe the physiological condition in mammals which is typically characterized by unregulated cell growth. Included in this definition are benign and malignant cancers as well as dormant tumors or micrometastases. Examples of cancer include, but are not limited to, carcinoma, lymphoma, blastoma, sarcoma and leukemia. More specific examples of such cancers include squamous cell carcinoma, lung cancer (including small cell lung cancer, non-small cell lung cancer, adenocarcinoma of the lung and squamous carcinoma of the lung), peritoneal cancer, hepatocellular cancer, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland carcinoma, renal cancer or cancer of the kidney, prostatic cancer, vulval cancer, thyroid cancer, cancer of the liver, and various types of head and neck cancers, as well as B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphomas, and Waldenstrom's macroglobulinemia), chronic lymphocytic leukemia (CLL), acute lymphoblastic leukemia (ALL), hairy cell leukemia, chronic myeloblastic leukemia, post-transplant lymphoproliferative disorder (PTLD), and abnormal vascular proliferation associated with phakomatoses, edema (such as associated with brain tumors) and Meigs syndrome.

Anti-TIGIT Antibody and Production Thereof

The term "anti-TIGIT antibody", "anti-TIGIT", "TIGIT antibody" or "TIGIT-binding antibody" refers to an antibody that is capable binding to a TIGIT protein or a fragment thereof with sufficient affinity such that the antibody can be used as a diagnostic and/or therapeutic agent targeting TIGIT.

Any suitable method for producing antibodies may be employed to produce the antibody disclosed herein. TIGIT in any suitable form may be used as an immunogen (antigen) for antibody production. By way of example and not limitation, any TIGIT variant or a fragment thereof may be used as an immunogen. In some embodiments, hybridoma cells that produce murine monoclonal anti-human TIGIT antibodies can be produced by methods well known in the art. These methods include, but are not limited to, hybridoma techniques originally developed by Kohler et al., (1975) (Nature 256:495-497). Preferably, mouse splenocytes are isolated and fused to a mouse myeloma cell line using PEG or by electrofusion according to standard schemes. Hybridoma cells secreting an antibody with TIGIT-inhibiting activity are then screened. The DNA sequence of the immunoglobulin variable region of the hybridoma cells disclosed herein may be determined using a degenerate primer-based PCR method.

Antibodies derived from rodents (e.g., mouse) may induce unwanted immunogenicity of the antibodies when used as therapeutic agents *in vivo.* Repeated use of these antibodies induces an immune response in the human body to therapeutic antibodies. Such immune responses result in at least a loss of therapeutic efficacy and, for severe cases, a potentially lethal allergic reaction. One method for reducing the immunogenicity of rodent antibodies includes producing chimeric antibodies, in which the mouse variable region is fused to the human constant region (Liu et al., (1987) Proc. Natl. Acad. Sci. USA 84:3439-43). However, the preservation of intact rodent variable region in a chimeric antibody can still induce deleterious immunogenicity in patients. Grafting of the complementarity determining region (CDR) loops of the rodent variable domain onto the human framework (i.e., humanization) has been used to further minimize rodent sequences (Jones et al., (1986) Nature 321:522; Verhoeyen et al., (1988) Science 239:1534).

In some embodiments, the chimeric or humanized antibodies disclosed herein can be prepared based on the sequences of the prepared murine monoclonal hybridoma antibodies. DNA encoding the immunoglobulin heavy and light chains can be obtained from a murine hybridoma of interest and engineered to comprise non-murine (e.g., human) immunoglobulin sequences using standard molecular biology techniques.

In some embodiments, for the chimeric TIGIT antibodies described herein, the chimeric heavy chains and the chimeric light chains can be obtained by operably linking the immunoglobulin heavy chain and light chain variable regions of hybridoma origin to human IgG constant regions respectively using methods known in the art (see, e.g., U.S. Pat. No.4,816,567 to Cabilly et al.). In some embodiments, the chimeric antibodies disclosed herein comprise constant regions which can be selected from any subtype of human IgG, such as IgG1, IgG2, IgG3 and IgG4, preferably IgG4.

In some embodiments, the chimeric TIGIT antibodies disclosed herein can be obtained by "mixing and matching" a chimeric light chain expression plasmid with a chimeric heavy chain expression plasmid to transfect expression cells. The TIGIT binding of such "mixed and matched" antibodies can be assayed using the above binding assays and other conventional binding assays (e.g., ELISA).

The precise amino acid sequence boundaries of the variable region CDRs of the antibodies disclosed herein can be determined using any of a number of well-known schemes, including Chothia based on the three-dimensional structure of antibodies and the topology of the CDR loops (Chothia et al., (1989) Nature 342:877-883; Al-Lazikani et al., "Standard conformations for the canonical structures of immunoglobulins", Journal of Molecular Biology, 273:927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures. The boundaries of the CDRs of the antibodies disclosed herein can be determined by one skilled in the art according to any scheme (e.g., different assignment systems or combinations) in the art.

It should be noted that the boundaries of the CDRs of the variable regions of the same antibody obtained based on different assignment systems may differ. That is, the CDR sequences of the variable regions of the same antibody defined under different assignment systems are different. Thus, when it comes to defining an antibody with a particular CDR sequence defined in the present invention, the scope of the antibody also encompasses antibodies whose variable region sequences comprise the particular CDR sequence but whose claimed CDR boundaries differ from the particular CDR boundaries defined in the present invention due to the application of different schemes (e.g., different assignment systems or combinations).

Antibodies with different specificities (i.e., different binding sites for different antigens) have different CDRs. However, although CDRs vary from antibody to antibody, only a limited number of amino acid positions within a CDR are directly involved in antigen binding. The smallest overlapping region can be determined using at least two of the Kabat, Chothia, AbM, Contact and North methods, thereby providing a "smallest binding unit" for antigen binding. The smallest binding unit may be a sub-portion of the CDR. As will be appreciated by those skilled in the art, the residues in the remainder of the CDR sequences can be determined by the antibody structure and protein folding. Thus, variants of any of the CDRs presented herein are also contemplated by the present invention. For example, in a variant of one CDR, the amino acid residue of the smallest binding unit may remain unchanged, while the remaining CDR residues defined according to Kabat or Chothia may be substituted by conservative amino acid residues.

For the humanized antibodies described herein, murine CDR regions can be inserted into human germline framework regions using methods known in the art. See U.S. Pat. No.5,225,539 to Winter et al. and U.S. Pat. Nos. 5,530,101, 5,585,089, 5,693,762 and 6,180,370 to Queen et al. Briefly, human germline IgG genes homologous to the cDNA sequence of the murine antibody variable regions were retrieved in the human immunoglobulin gene database of the NCBI (http://www.ncbi.nlm.nih.gov/igblast/) by the inventor, and in principle, humanization is achieved by grafting of the selected CDRs. However, CDR loop exchange still fails to uniformly produce an antibody with the same binding properties as the initial antibody. In humanized antibodies, changes in framework residues (FRs) (residues involved in CDR loop support) are often required to maintain the antigen-binding affinity. Briefly, the humanization comprises the following steps: A. comparing the gene sequence of each candidate antibody with the gene sequence of the human embryonic antibody to find out a sequence with high homology; B. analyzing and inspecting HLA-DR affinity, and selecting a human embryonic framework sequence with low affinity; and C. analyzing the framework amino acid sequences of the variable regions and their periphery by using a computer simulation technology and applying molecular docking to investigate their spatial and stereo combination modes. The key amino acid individuals that can interact with TIGIT and maintain the spatial framework in the gene sequence of the candidate antibodies were analyzed by calculating electrostatic force, Van der Waals' force, hydrophilicity and hydrophobicity, and entropy value, and grafted to the selected human embryonic gene framework. The amino acid sites of the framework regions which must be retained were mapped. After that, the humanized antibodies were synthesized.

In some embodiments, the anti-TIGIT antibodies or the antigen-binding fragments thereof disclosed herein include those antibodies having an amino acid sequence which has been mutated by amino acid deletion, insertion or substitution but still has at least 90%, 91%, 92%, 93%, 94%, 95%, 96%, 97%, 98% or 99% identity to the aforementioned antibodies (particularly in the CDR regions depicted in the aforementioned sequences). In some embodiments, when compared to the CDR regions depicted in a particular sequence of the present invention, the antibodies disclosed herein have no more than 1, 2, 3, 4 or 5 amino acid mutations (deletions, insertions or substitutions) in the CDR regions.

In some embodiments, the anti-TIGIT antibody or the antigen-binding fragment thereof described herein comprises 1 to 3 heavy chain complementarity determining regions selected from heavy chain complementarity determining regions HCDR1, HCDR2 and HCDR3, wherein the HCDR1 comprises an amino acid sequence identical to or having at least 90%, 91%, 92%, 93%, 94%, 95%, 96%, 97%, 98% or 99% identity to an amino acid sequence selected from SEQ ID NOs: 1 and 11, the HCDR2 comprises an amino acid sequence identical to or having at least 90%, 91%, 92%, 93%, 94%, 95%, 96%, 97%, 98% or 99% identity to an amino acid sequence selected from SEQ ID NOs: 2 and 12, and the HCDR3 comprises an amino acid sequence identical to or having at least 90%, 91%, 92%, 93%, 94%, 95%, 96%, 97%, 98% or 99% identity to an amino acid sequence selected from SEQ ID NOs: 3 and 13.

In some embodiments, the anti-TIGIT antibody or the antigen-binding fragment thereof described herein comprises heavy chain complementarity determining regions HCDR1, HCDR2 and HCDR3, wherein the HCDR1 comprises an amino acid sequence set forth in SEQ ID NO: 1, the HCDR2 comprises an amino acid sequence set forth in SEQ ID NO: 2, and the HCDR3 comprises an amino acid sequence set forth in SEQ ID NO: 3.

In some embodiments, the anti-TIGIT antibody or the antigen-binding fragment thereof described herein comprises heavy chain complementarity determining regions HCDR1, HCDR2 and HCDR3, wherein the HCDR1 comprises an amino acid sequence set forth in SEQ ID NO: 11, the HCDR2 comprises an amino acid sequence set forth in SEQ ID NO: 12, and the HCDR3 comprises an amino acid sequence set forth in SEQ ID NO: 13.

In some embodiments, the anti-TIGIT antibody or the antigen-binding fragment thereof described herein comprises 1 to 3 light chain complementarity determining regions selected from light chain complementarity determining regions LCDR1, LCDR2 and LCDR3, wherein the LCDR1 comprises an amino acid sequence identical to or having at least 90%, 91%, 92%, 93%, 94%, 95%, 96%, 97%, 98% or 99% identity to an amino acid sequence set forth in SEQ ID NO: 6 or 16, the LCDR2 comprises an amino acid sequence identical to or having at least 90%, 91%, 92%, 93%, 94%, 95%, 96%, 97%, 98% or 99% identity to an amino acid sequence set forth in SEQ ID NO: 7 or 17, and the LCDR3 comprises an amino acid sequence identical to or having at least 90%, 91%, 92%, 93%, 94%, 95%, 96%, 97%, 98% or 99% identity to an amino acid sequence set forth in SEQ ID NO: 8 or 18.

In some embodiments, the anti-TIGIT antibody or the antigen-binding fragment thereof described herein comprises light chain complementarity determining regions LCDR1, LCDR2 and LCDR3, wherein the LCDR1 comprises an amino acid sequence set forth in SEQ ID NO: 6, the LCDR2 comprises an amino acid sequence set forth in SEQ ID NO: 7, and the LCDR3 comprises an amino acid sequence set forth in SEQ ID NO: 8.

In some embodiments, the anti-TIGIT antibody or the antigen-binding fragment thereof described herein comprises light chain complementarity determining regions LCDR1, LCDR2 and LCDR3, wherein the LCDR1 comprises an amino acid sequence set forth in SEQ ID NO: 16, the LCDR2 comprises an amino acid sequence set forth in SEQ ID NO: 17, and the LCDR3 comprises an amino acid sequence set forth in SEQ ID NO: 18.

In some embodiments, the antibody or the antigen-binding fragment thereof described herein comprises heavy chain complementarity determining regions HCDR1, HCDR2 and HCDR3 and light chain complementarity determining regions LCDR1, LCDR2 and LCDR3, wherein the HCDR1 comprises an amino acid sequence set forth in SEQ ID NO: 1, the HCDR2 comprises an amino acid sequence set forth in SEQ ID NO: 2, the HCDR3 comprises an amino acid sequence set forth in SEQ ID NO: 3, the LCDR1 comprises an amino acid sequence set forth in SEQ ID NO: 6, the LCDR2 comprises an amino acid sequence set forth in SEQ ID NO: 7, and the LCDR3 comprises an amino acid sequence set forth in SEQ ID NO: 8.

In some embodiments, the antibody or the antigen-binding fragment thereof described herein comprises heavy chain complementarity determining regions HCDR1, HCDR2 and HCDR3 and light chain complementarity determining regions LCDR1, LCDR2 and LCDR3, wherein the HCDR1 comprises an amino acid sequence set forth in SEQ ID NO: 11, the HCDR2 comprises an amino acid sequence set forth in SEQ ID NO: 12, the HCDR3 comprises an amino acid sequence set forth in SEQ ID NO: 13, the LCDR1 comprises an amino acid sequence set forth in SEQ ID NO: 16, the LCDR2 comprises an amino acid sequence set forth in SEQ ID NO: 17, and the LCDR3 comprises an amino acid sequence set forth in SEQ ID NO: 18.

In some embodiments, the anti-TIGIT antibody or the antigen-binding fragment thereof described herein comprises a heavy chain variable region (VH), wherein the heavy chain variable region (VH) comprises an amino acid sequence identical to or having at least 90%, 91%, 92%, 93%, 94%, 95%, 96%, 97%, 98% or 99% identity to an amino acid sequence selected from SEQ ID NOs: 4, 14, 21, 23, 25, 27 and 29.

In some embodiments, the anti-TIGIT antibody or the antigen-binding fragment thereof described herein comprises a light chain variable region (VL), wherein the light chain variable region (VL) comprises an amino acid sequence identical to or having at least 90%, 91%, 92%, 93%, 94%, 95%, 96%, 97%, 98% or 99% identity to an amino acid sequence selected from SEQ ID NOs: 9, 19, 22, 24, 26, 28 and 30.

In some embodiments, the anti-TIGIT antibody or the antigen-binding fragment thereof described herein comprises a heavy chain variable region (VH) and a light chain variable region (VL), wherein the VH comprises an amino acid sequence identical to or having at least 90%, 91%, 92%, 93%, 94%, 95%, 96%, 97%, 98% or 99% identity to an amino acid sequence selected from SEQ ID NOs: 4, 14, 21, 23, 25, 27, and 29; and
the VL comprises an amino acid sequence identical to or having at least 90%, 91%, 92%, 93%, 94%, 95%, 96%, 97%, 98% or 99% identity to an amino acid sequence selected from SEQ ID NOs: 9, 19, 22, 24, 26, 28 and 30.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 4, and the VL comprises an amino acid sequence set forth in SEQ ID NO: 9.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 14, and the VL comprises an amino acid sequence set forth in SEQ ID NO: 19.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 21, and the VL comprises an amino acid sequence set forth in SEQ ID NO: 22.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 23, and the VL comprises an amino acid sequence set forth in SEQ ID NO: 24.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 25, and the VL comprises an amino acid sequence set forth in SEQ ID NO: 26.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 27, and the VL comprises an amino acid sequence set forth in SEQ ID NO: 28.

In some embodiments, the anti-TIGIT antibody or the antigen-binding fragment thereof described herein comprises a heavy chain variable region (VH) and a light chain variable region (VL), wherein the VH comprises an amino acid sequence set forth in SEQ ID NO: 29, and the VL comprises an amino acid sequence set forth in SEQ ID NO: 30.

In some embodiments, the anti-TIGIT antibody or the antigen-binding fragment thereof described herein is a murine antibody, a chimeric antibody, a humanized antibody or a fully human antibody.

In some embodiments, the anti-TIGIT antibody or the antigen-binding fragment thereof described herein is a complete antibody, a single-chain antibody, a Fab antibody, a Fab' antibody, a $(Fab')_2$ antibody or a bispecific (multispecific) antibody.

In some embodiments, the anti-TIGIT antibody or the antigen-binding fragment thereof described herein is of any IgG subtype, such as IgG1, IgG2, IgG3 or IgG4.

In some embodiments, one or more amino acid modifications may be introduced into an Fc region of an antibody provided herein, thus producing an Fc region variant. The Fc region variant may comprise human Fc region sequences (e.g., human IgG1, IgG2, IgG3 or IgG4Fc regions) which comprise amino acid modifications (e.g., substitutions) at one or more amino acid positions.

In some embodiments, antibodies modified by cysteine engineering may need to be produced, such as "sulfo-MAb", wherein one or more residues of the antibodies are substituted by cysteine residues.

In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

In some embodiments, the anti-TIGIT antibody or the antigen-binding fragment thereof described herein has one or more of the following properties: (1) specifically binding to human TIGIT protein; (2) cross-reacting with cynomolgus monkey TIGIT; (3) inhibiting binding of TIGIT to PVR and/or PVRL2; (4) inhibiting TIGIT-mediated activity signal transduction; (5) promoting activation of PD-1 axis antagonists on T cells; and (6) significantly inhibiting the growth of tumors when used alone or in combination with a PD-1 axis antagonist.

In some embodiments, the anti-TIGIT antibody or the antigen-binding fragment thereof described herein can specifically bind to TIGIT with a KD of about 1 nM or higher affinity (e.g., 1 nM-2 pM, 1 nM, 100 pM, 10 pM, or 2 pM). In one embodiment, the antibody disclosed herein that specifically binds to human TIGIT also cross-react with cynomolgus monkey TIGIT. As used herein, "cross-reactivity" refers to the ability of an antibody to react with homologous proteins from other species. Whether an antibody specifically binds to human TIGIT may be determined using any assay method known in the art. Examples of assay methods for determining binding affinity known in the art include surface plasmon resonance (e.g., BIACORE) or similar techniques (e.g., ForteBio).

In some embodiments, the anti-TIGIT antibody or the antigen-binding fragment thereof described herein has inhibitory activity, e.g., inhibiting the expression (e.g., the expression of TIGIT on the surface of cells), activity and/or signaling of TIGIT, or interfering with the interaction between TIGIT and PVR and/or PVRL2. The anti-TIGIT antibody provided herein completely or partially reduces or regulates the expression or activity of TIGIT upon binding to or interacting with TIGIT (e.g., human TIGIT). The biological function of TIGIT is completely, significantly or partially reduced or regulated upon the interaction between the antibodies and human TIGIT polypeptide and/or peptide. The antibody described herein is believed to be capable of completely inhibiting the expression or activity of TIGIT when the level of expression or activity of TIGIT is reduced by at least 95% (e.g., 96%, 97%, 98%, 99% or 100%) in the presence of the antibody as compared to the level of expression or activity of TIGIT in the absence of interaction (e.g., binding) with the antibody. The anti-TIGIT antibody described herein is believed to be capable of significantly inhibiting the expression or activity of TIGIT when the level of expression or activity of TIGIT is reduced by at least 50% (e.g., 55%, 60%, 75%, 80%, 85% or 90%) in the presence of the TIGIT antibody as compared to the level of expression or activity of TIGIT in the absence of binding to the TIGIT antibody. The antibody described herein is considered to be capable of partially inhibiting the expression or activity of TIGIT when the level of expression or activity of TIGIT is reduced by less than 95% (e.g., 10%, 20%, 25%, 30%, 40%, 50%, 60%, 75%, 80%, 85% or 90%) in the presence of the antibody as compared to the level of expression or activity of TIGIT in the absence of interaction (e.g., binding) with the antibody.

Expression of Antibodies

In one aspect, the present invention relates to a host cell comprising one or more expression vectors and a method for producing any of the antibodies or fragments thereof disclosed herein, and the method comprises: culturing the host cell, and purifying and isolating the antibody or the antigen-binding fragment thereof.

In one aspect, the present invention provides a nucleic acid encoding any of the aforementioned anti-TIGIT antibodies or fragments thereof. The nucleic acid can include a nucleic acid encoding an amino acid sequence of the light chain variable region and/or heavy chain variable region of the antibody, or a nucleic acid encoding an amino acid sequence of the light chain and/or heavy chain of the antibody.

In one embodiment, one or more vectors comprising the nucleic acid are provided. In one embodiment, the vector is an expression vector.

The present invention provides a mammalian host cell for expressing the recombinant antibodies disclosed herein, which includes a number of immortalized cell lines available from American Type Culture Collection (ATCC). These include, in particular, Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells, and many other cell lines. Mammalian host cells include human, mouse, rat, dog, monkey, pig, goat, cow, horse and hamster cells. Particularly preferred cell lines are selected by determining which cell line has high expression level.

In one embodiment, the present invention provides a method for preparing an anti-TIGIT antibody, wherein the method comprises: introducing an expression vector into a mammalian host cell, and culturing the host cell for a period of time sufficient to allow expression of the antibody in the host cell or more preferably to allow secretion of the antibody into a medium in which the host cell is grown, thereby producing the antibody. The antibody can be isolated from the medium using standard protein purification methods.

It is likely that antibodies expressed by different cell lines or in transgenic animals have different glycosylations from each other. However, all antibodies encoded by the nucleic acid molecules provided herein or comprising the amino acid sequences provided herein are integral parts of the present invention, regardless of the glycosylation of the antibody. Likewise, in certain embodiments, nonfucosylated antibodies are advantageous because they generally have more potent efficacy *in vitro* and *in vivo* than their fucosylated counterparts, and are unlikely to be immunogenic because their glycan structures are normal components of natural human serum IgG.

Pharmaceutical Composition and Pharmaceutical Formulation

The present invention provides a pharmaceutical composition which comprises one or more monoclonal antibodies or antigen-binding fragments thereof that bind to TIGIT. It should be understood that the anti-TIGIT antibodies or the antigen-binding fragments thereof or the pharmaceutical composition thereof provided herein can be integrated into a suitable carrier, an excipient and other reagents in a formulation for administration in combination, thus providing improved transfer, delivery, tolerance, etc.

The term "pharmaceutical composition" refers to a formulation which allows the biological activity of active ingredients contained therein to be present in an effective form and does not contain additional ingredients having toxicity unacceptable to a subject to which the formulation is administered.

The pharmaceutical formulation comprising the anti-TIGIT antibody or the antigen-binding fragment thereof described herein, preferably in the form of an aqueous solution or a lyophilized formulation, may be prepared by mixing the anti-TIGIT antibody or the antigen-binding fragment thereof disclosed herein having the desired purity with one or more optional pharmaceutical excipients (Remington's Pharmaceutical Sciences, 16th edition, Osol, A. Ed. (1980)).

The pharmaceutical composition or preparation disclosed herein can further comprise one or more additional active ingredients which are required for a specific indication being treated, preferably active ingredients having complementary activities that do not adversely affect one another. In some embodiments, the additional active ingredients are chemotherapeutic agents, immune checkpoint inhibitors, growth inhibitors, antibiotics or various known anti-tumor or anti-cancer agents, which are suitably present in combination in amounts that are effective for purpose intended. In some embodiments, the pharmaceutical composition disclosed herein also comprises a composition of a polynucleotide encoding the anti-TIGIT antibody or the antigen-binding fragment thereof.

Medical Use of Antibodies

In one aspect, the present invention relates to a method for administering to a subject an effective amount of any of the anti-TIGIT antibodies or the antigen-binding fragments thereof described herein, an immunoconjugate comprising the antibody or the antigen-binding fragment thereof or the pharmaceutical composition for inducing T cell- or NK cell-mediated anti-tumor activity or enhancing the immune response of the body.

In another aspect, the present invention relates to a method for administering to a subject an effective amount of any of the anti-TIGIT antibodies or the antigen-binding fragments thereof described herein, an immunoconjugate comprising the antibody or the antigen-binding fragment thereof or the pharmaceutical composition for treating or delaying various cancers, immune-related diseases and T cell dysfunctional diseases.

In another aspect, the present invention relates to the co-administration of an effective amount of one or more therapies (e.g., treatment modalities and/or additional therapeutic agents) to a subject. In some embodiments, the therapy includes surgical treatment and/or radiation therapy. In some embodiments, the additional therapeutic agent is selected from a chemotherapeutic agent and a PD-1 axis antagonist (e.g., an anti-PD-1 antibody, an anti-PD-Ll antibody or an anti-PD-L2 antibody).

In other aspects, the present invention provides use of the anti-TIGIT antibody or the antigen-binding fragment thereof described herein in producing or preparing a medicament for treating related diseases or disorders as mentioned above. The present invention also provides use of the anti-TIGIT antibody or the antigen-binding fragment thereof described herein and a PD-1 axis antagonist (e.g., an anti-PD-1 antibody, an anti-PD-Ll antibody or an anti-PD-L2 antibody) in producing or preparing a medicament for treating related diseases or disorders as mentioned above.

In certain embodiments, the methods and uses described herein also comprise: administering to the individual one or more therapies (e.g., treatment modalities and/or additional therapeutic agents). The antibody disclosed herein may be used alone or in combination with other therapeutic agents in a therapy. For example, the antibody may be co-administered with at least one additional therapeutic agent.

Methods for Diagnosis and Detection

In certain embodiments, any of the anti-TIGIT antibodies or the antigen-binding fragments thereof provided herein can be used to detect the presence of TIGIT in a biological sample. The term "detection" as used herein includes quantitative or qualitative detection. In certain embodiments, the biological sample is blood, serum, or other liquid samples of biological origin. In certain embodiments, the biological sample includes cells or tissues.

The present invention includes any combinations of the specific embodiments described. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entirety for all purposes.

The following abbreviations are used in this application:

RLU: relative luminescence unit;
BSA: bovine serum albumin.

Examples

Example 1: Recombinant Protein hTIGIT-ECD-mFC

A cDNA sequence encoding a TIGIT extracellular domain was amplified by a conventional PCR technique, and an amplified fragment was cloned into an autonomously constructed eukaryotic expression plasmid system (MX2-mFc, containing a murine IgG2a Fc domain) by employing a conventional cloning technique, wherein the eukaryotic expression plasmid system contained a puromycin screening system, so that a recombinant fusion protein expression plasmid hTIGIT-ECD-mFC was produced. The recombinant protein hTIGIT-ECD-mFC was expressed and purified by using an expression cell 293E.

The amino acid sequence of the human TIGIT extracellular domain (hTIGIT-ECD) is amino acids at positions 24-155 of NCBI Accession No. NM_173799.4, and is used for detecting immunogen and activity of the antibodies disclosed herein.

The sequence of the cynomolgus monkey TIGIT extracellular domain (cynoTIGIT-ECD) is NCBI Accession No. XP_015300912.1, and is used for detecting the activity of the antibodies disclosed herein.

Example 2. Preparation and Screening of Hybridoma Antibodies

Hybridoma antibodies were produced using standard molecular biological techniques. Briefly, the recombinant protein hTIGIT-ECD-mFC as an antigen was mixed with an equal amount of an immunoadjuvant, and 5 female Balb/c mice aged 6 weeks were immunized. Following the primary immunization, booster immunization was performed once the third week after the primary immunization and then once every two weeks, 6 immunizations in total. After the last booster immunization, mice with high anti-hTIGIT antibody titers in serum were selected as a source of immune cells for a cell fusion study. Spleen cells were isolated using a standard hybridoma technique and fused with cells of the murine myeloma cell line SP2/0 (ATCC) according to a conventional electroporation procedure (see the manual of BTX electroporation instrument). The fused cells were resuspended in DMEM complete medium (Corning) containing HAT and plated evenly in a 384-well plate containing mouse feeder cells.

Monoclonal hybridoma-secreted supernatants were identified based on initially desired antibody/antigen binding characteristics (such as binding specificity for TIGIT, ability to block the binding of TIGIT to PVR and blocking of TIGIT-mediated bioactivity), with the supernatant antibodies secreted by hybridomas 2018 and 23O20 used for further characterization.

Example 3: Effect of Murine Anti-TIGIT Antibodies on Activity of T Cells

T cell activation was achieved by stimulating T cell receptors (TCRs) that recognize specific peptides presented by major histocompatibility complex class I or class II proteins on antigen-presenting cells (APCs). Then the activated TCRs initiated a cascade of signaling events that could be monitored by reporter genes driven by transcription factors (such as activator-protein-l (AP-l), nuclear factor of activated T cells (NFAT), or nuclear factor κ-light-chain-enhancer of activated B cells (NFκb)). T cell responses were regulated by the composition or induction of engagement of expressed co-receptors on T cells. Programmed cell death protein (PD1) and TIGIT were negative regulators of T cell activity. PD-1 and TIGIT interacted with their ligands (PD-L1) and PVR (and/or PVRL2), respectively, expressed on target cells including APC or cancer cells, which resulted in the delivery of an inhibitory signal by recruitment of phosphatase to the TCR signalosome, thereby producing inhibition of positive signaling. T cell signaling induced by the interaction between APC and T cells was measured by constructing two engineered stable cell lines, Jurkat cells (Jurkat/NFAT-Luc/hPD-1-hTIGIT) and CHO cells (CHO/hPD-L1-hPVRL2).

Specifically, CHO cells stably expressing hPD-L1/hPVRL2 were plated into a 96-well plate at $5\times10^4$ cells/well and incubated overnight at 37 °C and 7% $CO_2$. After cell supernatant was removed, 40 μL of anti-TIGIT antibody (2018, 23O20 or MBSA43) dilution (an initial concentration of 10 ug/mL, 3-fold titration) was added into each well, and/or 40 μL of JS001 (0.5 ug/mL) was added in advance. 40 μL of Jurkat reporter cells capable of continuously expressing hPD-1/hTIGIT/NFAT-luciferase were added, with the cells being $1\times10^5$ in total. After 6 hours of incubation at 37 °C and 7% $CO_2$, a luciferase reagent was added, and luminescence values were detected by a microplate reader.

MBSA43 was a marketed anti-human TIGIT antibody purchased from ThermoFisher (Cat. No.: 16-9500-82).

JS001 was an anti-PD-1 antibody (CN201310258289, antibody 38) independently developed by Junshi Biosciences.

Data were analyzed using GraphPad Prism 5 to calculate $EC_{50}$ values for activation of T cells by the TIGIT antibodies and thereby evaluate the effect of the anti-TIGIT antibodies alone or in combination with the anti-PD-1 antibody (JS001) on the activity of the T cells. See FIG. 1 and Table 1 below for details.

Table 1. Effect of anti-TIGIT antibodies alone or in combination with anti-PD-1 antibody (JS001) on activity of T cells

| Group | Antibody | RLU fold change | $EC_{50}$ |
|---|---|---|---|
| Group 1 | 2018 +JS001 | 3.3 | 65.47 |
| Group 2 | 2018 alone | 6.3 | 51.78 |
| Group 3 | 23O20+JS001 | 2.3 | 180.9 |
| Group 4 | 23O20 alone | 2.3 | 195.5 |
| Group 5 | MBSA43+JS001 | 2.2 | 164.3 |
| Group 6 | MBSA43 alone | 2.2 | 179.7 |
| Group 7 | mIgG+JS001 | 1.2 | ~ 11641 |
| Group 8 | mIgG alone | 1.2 | ~ 13058 |
| mIgG: a negative control antibody. | | | |

The results show that:

(1) the antibodies 2018 and 23O20 can significantly promote the activity of the T cells;
(2) the effect of the antibody 2018 in activating the T cells is remarkably better than that of the marketed antibody MBSA43;
(3) the antibodies 2018, 23O20 and MBSA43 all have a synergistic effect on T cell activation when used in combination with the anti-PD-1 antibody JS001.

Example 4: Construction and Expression of Chimeric Antibodies

The DNA sequences of the antibody variable regions expressed by the hybridomas 2018 and 23O20 were determined using a degenerate primer PCR-based method.

Nucleotide sequences and amino acid sequences of 2O18 and 23O20 are shown in Table 2.

Table 2. Amino acid sequences of variable regions of hybridoma antibodies

| Hybridoma antibodies | Variable region | Amino acid sequence | Nucleotide sequence |
|---|---|---|---|
| 2018 | VH | SEQ ID NO: 4 | SEQ ID NO: 5 |
| | VL | SEQ ID NO: 9 | SEQ ID NO: 10 |
| 23020 | VH | SEQ ID NO: 14 | SEQ ID NO: 15 |
| | VL | SEQ ID NO: 19 | SEQ ID NO: 20 |

A human IgG4 heavy chain constant region Fc fragment (SEQ ID NO: 35) and a light chain constant region were cloned from human blood cells (Beijing Blood Institute) and ligated with pCDNA3.1 plasmid for engineering. The aforementioned heavy chain and light chain variable region sequence fragments were synthesized by GenScript. The heavy and light chains were cleaved by Bspq I and then ligated to the correspondingly engineered pCDNA3.1 plasmid. The expression plasmids for IgG4 chimeric heavy chains (ch-HC) or light chains (ch-LC) were verified by sequencing. The aforementioned different expression plasmids for the chimeric heavy and light chains were paired to transfect expression cells, producing 4 chimeric antibodies numbered ch1 to ch4 (see Table 3).

Table 3. Chimeric antibody light/heavy chain sources

| HC<br>LC | 2O18-ch-HC | 23O20-ch-HC |
|---|---|---|
| 2O18-ch-LC | ch1 | ch3 |
| 23O20-ch-LC | ch2 | ch4 |

Example 5: Detection of Binding Activity of Chimeric Anti-TIGIT Antibodies to Antigen TIGIT by ELISA

1 μg/mL of human TIGIT-ECD-mFc was immobilized on a 96-well plate and incubated for 60-90 minutes at a constant temperature of 37 °C. The solution in wells was then discarded, and the wells were washed 3 times with a washing buffer and blocked for 60 minutes with PBS containing 2% BSA. After the plate was washed 3 times with the washing buffer, chimeric antibody dilutions at different concentrations were added. The antibodies were incubated at 37 °C for 60 minutes, and then the plate was washed 3 times with the washing buffer and added with 5000-fold diluted anti-IgG4-HRP. The system was incubated at 37 °C for 30 minutes, and then washed three times with the washing buffer and added with 100 μL of TMB substrate for color development. The system was incubated at room temperature for 30 minutes, and the reaction was then terminated with 100 μL of 2 M hydrochloric acid solution. The absorbance at 450 nm was measured by a plate reader.

As shown in FIG. 2, the chimeric antibodies ch1 and ch4 bind to human TIGIT with higher specificity, and the $EC_{50}$ values of the chimeric antibodies are 32.49 ng/mL and 8.932 ng/mL, respectively.

Example 6: Effect of Chimeric Anti-TIGIT Antibodies on Activity of T Cells

According to the experimental principle and method as described in Embodiment 3, the effect of the chimeric antibodies ch1 and ch4 on the activity of T cells was detected using a luciferase reporter gene experiment (luciferase assay).

As shown in FIG. 3, the chimeric antibodies ch1 and ch4 can significantly enhance fluorescence signals, i.e., promoting the activation of T blood cells, and the $EC_{50}$ values of the chimeric antibodies are 76.64 ng/mL and 103.9 ng/mL, respectively.

Example 7. Humanization of Antibodies

For humanization of antibodies, human IgG genes homologous to the cDNA sequence of the murine antibody variable regions were retrieved in the human immunoglobulin gene database of the NCBI (http://www.ncbi.nlm.nih.gov/igblast/). The amino acid sequences and precise boundaries of the variable region CDRs were then defined by the Kabat numbering system or the IMGT numbering system. The CDR sequences of the murine antibody variable regions defined by the IMGT are shown in Table 4. Human IGVH and IGVk with high homology to the variable regions of the murine antibody were selected as templates for humanization and were humanized by CDR grafting. Briefly, the humanization comprises the following steps: A. comparing the gene sequence of each candidate antibody with the gene sequence of the human embryonic antibody to find out a sequence with high homology; B. analyzing and inspecting HLA-DR affinity, and selecting a human embryonic framework sequence with low affinity; and C. analyzing the framework amino acid sequences of the variable regions and their periphery by using a computer simulation technology and applying molecular docking to investigate their spatial and stereo combination modes. The key amino acid individuals that can interact with TIGIT and maintain the spatial framework in the gene sequence of the candidate antibodies were analyzed by calculating electrostatic force, Van der Waals' force, hydrophilicity and hydrophobicity, and entropy value, and grafted to the selected human embryonic gene framework. The amino acid sites of the framework regions which must be retained were mapped. After that, the humanized antibodies were synthesized. Based on the above factors, a total of 81 humanized antibodies were designed for antibody activity screening. The amino acid sequence information of antibodies hu3, hu20, hu62, hu69 and hu81 is shown in Table 5, and the full-length amino acid sequence information of the antibodies hu3 and hu20 is shown in Table 6.

Table 4: IMGT-defined CDRs

| Antibody / Domain | 2O18 | 23O20 |
|---|---|---|
| VH | SEQ ID NO: 4 | SEQ ID NO: 14 |
| HCDR1 | SEQ ID NO: 1 | SEQ ID NO: 11 |
| HCDR2 | SEQ ID NO: 2 | SEQ ID NO: 12 |
| HCDR3 | SEQ ID NO: 3 | SEQ ID NO: 13 |
| VL | SEQ ID NO: 9 | SEQ ID NO: 19 |
| LCDR1 | SEQ ID NO: 6 | SEQ ID NO: 16 |
| LCDR2 | SEQ ID NO: 7 | SEQ ID NO: 17 |
| LCDR3 | SEQ ID NO: 8 | SEQ ID NO: 18 |

Table 5: Amino acid sequences of humanized antibodies (CDRs/variable regions)

| Humanized antibody | hu3 | hu20 | hu62 | hu69 | hu81 |
|---|---|---|---|---|---|
| HCDR1 | SEQ ID NO: 1 | SEQ ID NO: 1 | SEQ ID NO: 11 | SEQ ID NO: 11 | SEQ ID NO: 11 |
| HCDR2 | SEQ ID NO: 2 | SEQ ID NO: 2 | SEQ ID NO: 12 | SEQ ID NO: 12 | SEQ ID NO: 12 |
| HCDR3 | SEQ ID NO: 3 | SEQ ID NO: 3 | SEQ ID NO: 13 | SEQ ID NO: 13 | SEQ ID NO: 13 |
| LCDR1 | SEQ ID NO: 6 | SEQ ID NO: 6 | SEQ ID NO: 16 | SEQ ID NO: 16 | SEQ ID NO: 16 |
| LCDR2 | SEQ ID NO: 7 | SEQ ID NO: 7 | SEQ ID NO: 17 | SEQ ID NO: 17 | SEQ ID NO: 17 |
| LCDR3 | SEQ ID NO: 8 | SEQ ID NO: 8 | SEQ ID NO: 18 | SEQ ID NO: 18 | SEQ ID NO: 18 |
| VH | SEQ ID NO: 21 | SEQ ID NO: 23 | SEQ ID NO: 25 | SEQ ID NO: 27 | SEQ ID NO: 29 |
| VL | SEQ ID NO: 22 | SEQ ID NO: 24 | SEQ ID NO: 26 | SEQ ID NO: 28 | SEQ ID NO: 30 |

Table 6: Full-length amino acids of humanized antibodies

| Humanized antibody | HC | LC |
|---|---|---|
| hu3 | SEQ ID NO: 31 | SEQ ID NO: 32 |
| hu20 | SEQ ID NO: 33 | SEQ ID NO: 34 |

Example 8: Detection of Binding Specificity of Humanized Antibodies for Human TIGIT by ELISA

The binding specificity of the humanized antibodies for human TIGIT was detected by conventional ELISA. Namely, 0.5 μg/mL of human TIGIT-ECD-mFc was immobilized on a 96-well plate and incubated for 60-90 minutes at a constant temperature of 37 °C. The solution in wells was then discarded, and the wells were washed 3 times with a washing buffer and blocked for 60 minutes with PBS containing 2% BSA. After the plate was washed 3 times with the washing buffer, humanized antibody dilutions at different concentrations were added. The antibodies were incubated at 37 °C for 60 minutes, and then the plate was washed 3 times with the washing buffer and added with 5000-fold diluted anti-IgG4-HRP. The system was incubated at 37 °C for 30 minutes, and then washed three times with the washing buffer and added with 100 μL of TMB substrate for color development. The system was incubated at room temperature for 30 minutes, and the reaction was then terminated with 100 μL of 2 M hydrochloric acid solution. The absorbance at 450 nm was measured by a plate reader.

As shown in FIG. 4, the antibodies hu3, hu20, hu62, hu69 and hu81 bind to human TIGIT with higher specificity, and the $EC_{50}$ values of the antibodies are 23.20 ng/mL, 37.84 ng/mL, 8.60 ng/mL, 10.66 ng/mL and 9.83 ng/mL, respectively; the TIGIT-binding specificity of the antibodies hu3, hu62, hu69 and hu81 is significantly better than that of the control antibody etigilimab.

Example 9: Effect of Humanized Anti-TIGIT Antibodies on Activity of T Cells

According to the experimental principle and method as described in Embodiment 3, the effect of the humanized anti-TIGIT antibodies hu3, hu20, hu62, hu69 and hu81 on the activity of T cells was detected using a luciferase reporter gene experiment (luciferase assay).

As shown in FIG. 5, the antibodies hu3, hu20, hu62, hu69 and hu81 can remarkably enhance fluorescence signals, i.e., promoting the activation of T blood cells, and the $EC_{50}$ values of the antibodies are 139.9 ng/mL, 42.39 ng/mL, 109.4 ng/mL, 102.7 ng/mL and 88.43 ng/mL, respectively; the effect of the antibodies hu3, hu20, hu62, hu69 and hu81 in promoting the activation of T blood cells is significantly better than that of the control antibody etigilimab.

Example 10: Detection of Effect of Antibodies in Blocking Binding of hTIGIT to Its Ligand hPVR by FACS

The effect of the antibodies in blocking the binding of hTIGIT to its ligand hPVR was detected by a competitive flow cytometry-based assay (FACS). Briefly, antibody dilutions at different concentrations (an initial concentration of 30 μg/mL, 3-fold titration) were each mixed with PVR-mFC (1 ug/mL, 50 μL) and incubated for 30 minutes at room temperature. The mixture was then incubated with cell suspension (stable 293F-hPVR cell line, $2.5\times10^4$ cells/well) for 15 minutes at 37 °C. After 3 times of elution with PBS, 100 μL of goat anti-human IgG-PE antibody was added, and the system was incubated for 30 minutes in the dark. After 3 times of elution with PBS, detection was performed by FACS. Viable cells were gated on the basis of FSC/SSC, and their geometric mean fluorescence intensity was measured.

As shown in FIG. 6, the humanized antibodies disclosed herein can effectively block the binding of TIGIT to PVR on the cell surface.

Example 11: Detection of Binding of Humanized Antibodies to TIGIT of Different Species by BIACORE

The affinity of the antibodies disclosed herein for TIGIT and the binding kinetics thereof were detected using Biacore T200 (GE). An S series CM5 chip (GE) was first loaded onto the instrument, and 40 μg/mL of goat anti-human Fc fragment antibody (Jackson ImmuneResearch) dissolved in sodium acetate-acetic acid buffer (pH 5.0) was ready to be coupled to the chip surface. The buffer used for the detection of antibody-antigen binding was HBS-EP+ from GE Healthcare. The antibodies hu3 and hu20 were each diluted to 8 μg/mL and captured on the chip surface. The antigen hTIGIT Fc was diluted to 20 nM and injected onto the chip surface at a flow rate of 30 μL/min for 180 s, and binding dissociation signals of the antibodies and the antigen were detected. The resulting data were analyzed with a 1:1 binding model by using Biacore T200 Evaluation Software 3.0. The kinetic constants of the binding of the antibodies to the antigen, namely, the association rate constant ka (1/Ms), the dissociation rate constant kd (1/s) and the equilibrium dissociation constant $K_D$ (M), were obtained by fitting, and the results are shown in Table 7 below.

Table 7: Affinity of antibodies for TIGIT-Fc

| Antibody | Antigen | ka (1/Ms) | kd (1/s) | $K_D$ (M) |
|---|---|---|---|---|
| hu3 | Cyno TIGIT-Fc | 2.11E+05 | 3.00E-04 | 1.42E-09 |
| hu20 | Cyno TIGIT-Fc | 2.26E+05 | 3.29E-04 | 1.45E-09 |
| hu3 | hTIGIT-Fc | 9.35E+05 | 1.21E-04 | 1.30E-10 |
| hu20 | hTIGIT-Fc | 7.77E+05 | 1.63E-04 | 2.09E-10 |

The results show that the antibodies disclosed herein have high affinity for both human TIGIT and cynomolgus monkey TIGIT, and the $K_D$ values of the antibodies for human TIGIT are as low as 0.13 nM.

Example 12: Inhibition of Tumor Growth in Mice by Humanized Antibodies

The study relates to the establishment of MC38 colon cancer animal models of B-hPD-1/hTIGIT humanized mice (purchased from Biocytogen Jiangsu Co., Ltd.; female) and synergistic anti-tumor effect of the anti-TIGIT antibodies and the anti-PDI antibody administered in combination.

The B-hPD-1/hTIGIT humanized mice were each subcutaneously inoculated, in the right dorsal side, with 0.1 mL of MC38 cells resuspended in PBS at a concentration of $5\times10^5$ cells/0.1 mL. When the average tumor volume reached 80-100 $mm^3$, appropriate mice were selected according to the tumor volume and body weight of the mice and evenly distributed into 8 experimental groups, 8 mice in each group, and administration was started on the day of grouping. The specific administration regimen is shown in Table 8 below.

Table 8: Administration regimen

| Group | Administered antibody | Dosage (mg/kg)[a] | Route of administration | Frequency of administration[b] | Number of administrations |
|---|---|---|---|---|---|
| 1 | KLH hIgG4 | 10 | i.p | BIW | 6 |
| 2 | JS001 | 0.3 | i.p | BIW | 6 |
| 3 | hu20 | 1 | i.p | BIW | 6 |
| 4 | hu20 | 3 | i.p | BIW | 6 |
| 5 | hu20 | 10 | i.p | BIW | 6 |
| 6 | JS001+hu20 | 0.3+1 | i.p | BIW | 6 |
| 7 | JS001+hu20 | 0.3+3 | i.p | BIW | 6 |
| 8 | JS001+hu20 | 0.3+10 | i.p | BIW | 6 |
| Note: a: the administration volume is calculated at 10 μL/g according to the body weight of the experimental animal;<br>b: BIW means administering twice a week;<br>i.p: intraperitoneal injection;<br>KLH hIgG4: a negative control antibody;<br>JS001: an anti-PD-1 antibody (CN2013102582892, antibody 38) independently developed by Junshi Biosciences. | | | | | |

Throughout the study, tumor volume and animal body weight were measured twice a week from day 6 (before administration) for 3 consecutive weeks. The long diameter (L) and short diameter (W) of each tumor were measured using a vernier caliper, and the tumor volume (V) was calculated using the following formula: $V = L \times W^2/2$. The tumor volume over time of the mice in various groups was plotted. Statistical significance was determined using analysis of variance (ANOVA). A P value below 0.05 was considered statistically significant in all analyses.

At the end of the 26-day experiment, the mice were euthanized. Tumor tissues were isolated, photographed and weighed, tumor weight and volume (final tumor volume) of each group of mice were measured, and relative tumor growth inhibition rates (TGI (%)) were calculated.

Results:

(1) As shown in FIG. 7, in terms of monotherapy, after 3 weeks of administration, compared to administration group 1 (KLH hIgG4; 10 mg/kg), administration group 2 (JS001; 0.3 mg/kg), administration group 3 (hu20; 1 mg/kg), administration group 4 (hu20; 3 mg/kg) and administration group 5 (hu20; 10 mg/kg) significantly inhibit tumor growth in the MC38 tumor-bearing mice as the tumor volume is reduced and tumor growth is slowed down as well. In terms of co-administration, the co-administration groups have a more remarkable inhibiting effect on tumor growth in the MC38 tumor-bearing mice than the monotherapy groups (group 6 vs group 3/group 2; group 7 vs group 4/group 2; group 8 vs group 5/group 2), that is, the co-administration of the anti-TIGIT monoclonal antibody hu20 disclosed herein and the anti-PD-1 monoclonal antibody JS001 shows a good synergistic anti-tumor effect.
(2) The present invention also calculates the relative tumor growth inhibition rate in each group of mice at the end of the study on day 26 (after tumor implantation). The calculation formula for the relative tumor growth inhibition rate is as follows:

$$\text{Relative tumor growth inhibition rate TGI (\%)} = [1 - (T_i - T_0)/(V_i - V_0)] \times 100\%$$

where $T_i$ - $T_0$ = final tumor volume of the administration group after administration - tumor volume of the administration group before administration, and $V_i$ - Vo = final tumor volume of the control group after administration - tumor volume of the control group before administration (tumor volume before administration on day 6).

The calculation results are shown in Table 9 below:

Table 9: Effect of anti-TIGIT antibodies on tumor tissue growth in tumor-bearing mice ($mm^3$, mean±SD, n = 8)

| Group | Administered antibody, dosage | Mean tumor volume ($mm^3$, mean±SD, n = 8) upon completion of the experiment | TGI(%) |
|---|---|---|---|
| 1 | KLH hIgG4; 10mg/kg | 2562±529 | N/A |
| 2 | JS001; 0.3mg/kg | 1713±876 | 34.3% |
| 3 | hu20; 1mg/kg | 1739±956 | 33.2% |
| 4 | hu20; 3mg/kg | 1540±809 | 41.3% |
| 5 | hu20; 10mg/kg | 1555±549 | 40.7% |
| 6 | JS001 +hu20; 0.3 mg/kg +1 mg/kg | 1153±617 | 56.9% |
| 7 | JS001 +hu20; 0.3 mg/kg +3mg/kg | 1101±712 | 59.0% |
| 8 | JS001 +hu20; 0.3 mg/kg +10mg/kg | 990±609 | 63.5% |
| Mean±SD: mean±standard deviation | | | |

The results show that at the final stage of the experiment (on day 26 after tumor implantation), the relative tumor growth inhibition rate TGI (%) of the groups administered with the anti-TIGIT antibody hu20 in combination with the anti-PD-1 antibody JS001 was significantly higher than that of the groups administered with hu20 or JS001 alone (group 6 vs group 3/group 2; group 7 vs group 4/group 2; group 8 vs group 5/group 2).

## Claims

1. An isolated anti-TIGIT antibody or an antigen-binding fragment thereof, wherein the anti-TIGIT antibody or the antigen-binding fragment thereof comprises:

(1) 1 to 3 heavy chain complementarity determining regions selected from heavy chain complementarity determining regions HCDR1, HCDR2 and HCDR3, wherein the HCDR1 comprises an amino acid sequence identical to or having at least 90% identity to an amino acid sequence set forth in SEQ ID NO: 1 or 11, the HCDR2 comprises an amino acid sequence identical to or having at least 90% identity to an amino acid sequence set forth in SEQ ID NO: 2 or 12, and the HCDR3 comprises an amino acid sequence identical to or having at least 90% identity to an amino acid sequence set forth in SEQ ID NO: 3 or 13; and/or
(2) 1 to 3 light chain complementarity determining regions selected from light chain complementarity determining regions LCDR1, LCDR2 and LCDR3, wherein the LCDR1 comprises an amino acid sequence identical to or having at least 90% identity to an amino acid sequence set forth in SEQ ID NO: 6 or 16, the LCDR2 comprises an amino acid sequence identical to or having at least 90% identity to an amino acid sequence set forth in SEQ ID NO: 7 or 17, and the LCDR3 comprises an amino acid sequence identical to or having at least 90% identity to an amino acid sequence set forth in SEQ ID NO: 8 or 18.

2. The antibody or the antigen-binding fragment thereof according to claim 1, wherein the antibody or the antigen-binding fragment thereof comprises heavy chain complementarity determining regions HCDR1, HCDR2 and HCDR3 and light chain complementarity determining regions LCDR1, LCDR2 and LCDR3, wherein:

(1) the HCDR1 comprises an amino acid sequence set forth in SEQ ID NO: 1, the HCDR2 comprises an amino acid sequence set forth in SEQ ID NO: 2, the HCDR3 comprises an amino acid sequence set forth in SEQ ID NO: 3, the LCDR1 comprises an amino acid sequence set forth in SEQ ID NO: 6, the LCDR2 comprises an amino acid sequence set forth in SEQ ID NO: 7, and the LCDR3 comprises an amino acid sequence set forth in SEQ ID NO: 8; or
(2) the HCDR1 comprises an amino acid sequence set forth in SEQ ID NO: 11, the HCDR2 comprises an amino acid sequence set forth in SEQ ID NO: 12, the HCDR3 comprises an amino acid sequence set forth in SEQ ID NO: 13, the LCDR1 comprises an amino acid sequence set forth in SEQ ID NO: 16, the LCDR2 comprises an amino acid sequence set forth in SEQ ID NO: 17, and the LCDR3 comprises an amino acid sequence set forth in SEQ ID NO: 18.

**3.** The antibody or the antigen-binding fragment thereof according to claim 1 or 2, wherein the antibody comprises a heavy chain variable region (VH) and a light chain variable region (VL), wherein:

the VH comprises an amino acid sequence identical to or having at least 90%, 91%, 92%, 93%, 94%, 95%, 96%, 97%, 98% or 99% identity to any amino acid sequence selected from SEQ ID NOs: 4, 14, 21, 23, 25, 27 and 29; and/or
the VL comprises an amino acid sequence identical to or having at least 90%, 91%, 92%, 93%, 94%, 95%, 96%, 97%, 98% or 99% identity to any amino acid sequence selected from SEQ ID NOs: 9, 19, 22, 24, 26, 28 and 30.

**4.** The antibody or the antigen-binding fragment thereof according to claim 3, wherein the antibody comprises a heavy chain variable region (VH) and a light chain variable region (VL), and the antibody is selected from:

(1) an antibody in which a VH comprises an amino acid sequence set forth in SEQ ID NO: 4 and a VL comprises an amino acid sequence set forth in SEQ ID NO: 9;
(2) an antibody in which a VH comprises an amino acid sequence set forth in SEQ ID NO: 14 and a VL comprises an amino acid sequence set forth in SEQ ID NO: 19;
(3) an antibody in which a VH comprises an amino acid sequence set forth in SEQ ID NO: 21 and a VL comprises an amino acid sequence set forth in SEQ ID NO: 22;
(4) an antibody in which a VH comprises an amino acid sequence set forth in SEQ ID NO: 23 and a VL comprises an amino acid sequence set forth in SEQ ID NO: 24;
(5) an antibody in which a VH comprises an amino acid sequence set forth in SEQ ID NO: 25 and a VL comprises an amino acid sequence set forth in SEQ ID NO: 26;
(6) an antibody in which a VH comprises an amino acid sequence set forth in SEQ ID NO: 27 and a VL comprises an amino acid sequence set forth in SEQ ID NO: 28; and
(7) an antibody in which a VH comprises an amino acid sequence set forth in SEQ ID NO: 29 and a VL comprises an amino acid sequence set forth in SEQ ID NO: 30.

**5.** The antibody or the antigen-binding fragment thereof according to any of claims 1 to 4, wherein an amino acid sequence of a heavy chain of the antibody is set forth in SEQ ID NO: 31 or 33, and/or an amino acid sequence of a light chain of the antibody is set forth in SEQ ID NO: 32 or 34.

**6.** The antibody or the antigen-binding fragment thereof according to claim 5, wherein the amino acid sequence of the heavy chain of the antibody is set forth in SEQ ID NO: 31, and the amino acid sequence of the light chain of the antibody is set forth in SEQ ID NO: 32; or the amino acid sequence of the heavy chain of the antibody is set forth in SEQ ID NO: 33, and the amino acid sequence of the light chain of the antibody is set forth in SEQ ID NO: 34.

**7.** The antibody or the antigen-binding fragment thereof according to any of claims 1 to 4, wherein the antibody is a murine antibody, a chimeric antibody, a humanized antibody or a fully human antibody.

**8.** The antibody or the antigen-binding fragment thereof according to any of claims 1 to 4, wherein the antibody or the antigen-binding fragment thereof is a complete antibody, a single-chain antibody, a Fab antibody, a Fab' antibody, a (Fab')2 antibody or a bispecific (multispecific) antibody.

**9.** The antibody or the antigen-binding fragment thereof according to any of claims 1 to 4, wherein the antibody is of any IgG subtype, such as IgG1, IgG2, IgG3 or IgG4.

**10.** An isolated nucleic acid molecule, encoding the antibody or the antigen-binding fragment thereof according to any of claims 1 to 9.

**11.** A recombinant or expression vector, comprising one or more nucleic acid sequences according to claim 10, wherein the vector is suitable for recombinant production of the antibody or the antigen-binding fragment thereof according to any of claims 1 to 9.

**12.** A host cell, comprising one or more recombinant or expression vectors according to claim 11 or nucleic acid molecules according to claim 10, and/or expressing the antibody or the anti-binding fragment thereof according to any of claims 1 to 9.

**13.** A pharmaceutical composition, comprising a composition of the antibody or the antigen-binding fragment thereof according to any of claims 1 to 9 and a pharmaceutically acceptable carrier or excipient.

**14.** The pharmaceutical composition according to claim 13, further comprising a PD-1 axis antagonist.

**15.** A method for treating cancer in a subject, comprising: administering to the subject an effective amount of the anti-TIGIT antibody or the antigen-binding fragment thereof according to any of claims 1 to 9 or the pharmaceutical composition according to any of claims 13 to 14.

**16.** The method according to claim 15, further comprising: co-administering to the subject one or more therapies comprising surgical treatment and/or radiation therapy, and/or administering one or more other therapeutic agents comprising a chemotherapeutic agent, a PD-1 axis antagonist and an anti-angiogenic agent.

**17.** The method according to claim 16, wherein the therapeutic agent is a PD-1 axis antagonist.

**18.** Use of the antibody or the antigen-binding fragment thereof according to any of claims 1 to 9, the nucleic acid molecule according to claim 10, the vector according to claim 11, the host cell according to claim 12 or the pharmaceutical composition according to claim 14 in preparing a medicament for treating and/or preventing a TIGIT-mediated disease or disorder, wherein preferably, the disease or disorder is cancer.

**19.** A method for producing the antibody or the antigen-binding fragment thereof according to any of claims 1 to 9, comprising: culturing the host cell according to claim 12, and isolating the antibody or the antigen-binding fragment thereof from the culture.

**20.** Use of a combination of the antibody or the antigen-binding fragment thereof according to any of claims 1 to 9 and a PD-1 axis antagonist in preparing a medicament for treating cancer in a subject, wherein preferably, the anti-PD-1 axis antagonist is selected from an anti-PD-1 antibody, an anti-PD-Ll antibody and an anti-PD-L2 antibody.

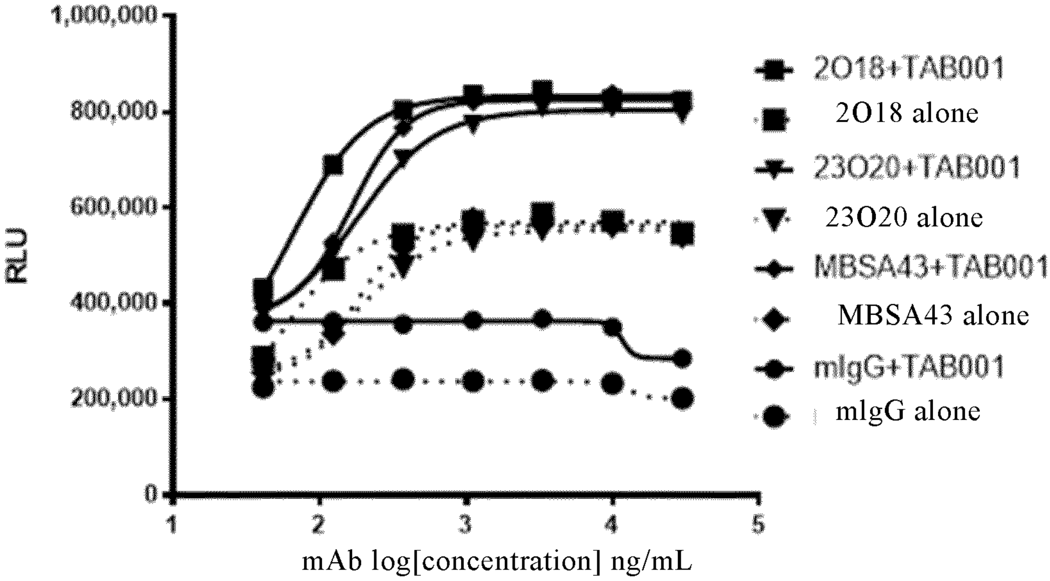


FIG. 1

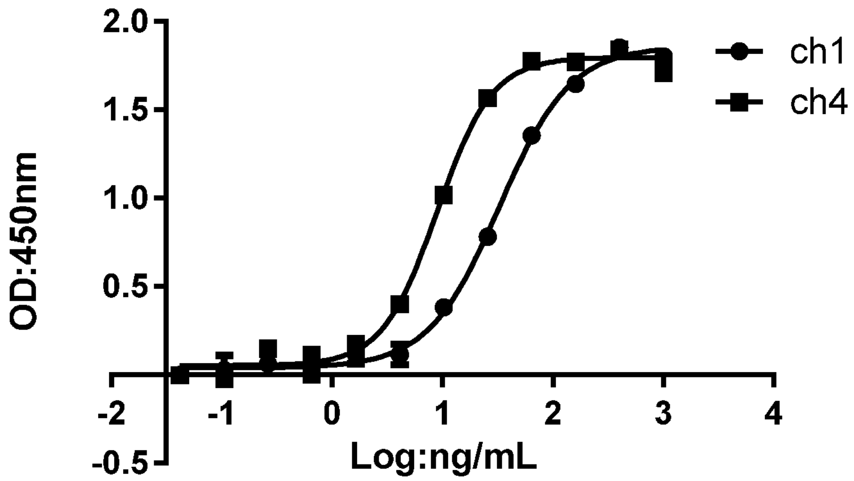


| | ch1 | ch4 |
|---|---|---|
| EC50 | 32.49 | 8.932 |

FIG. 2

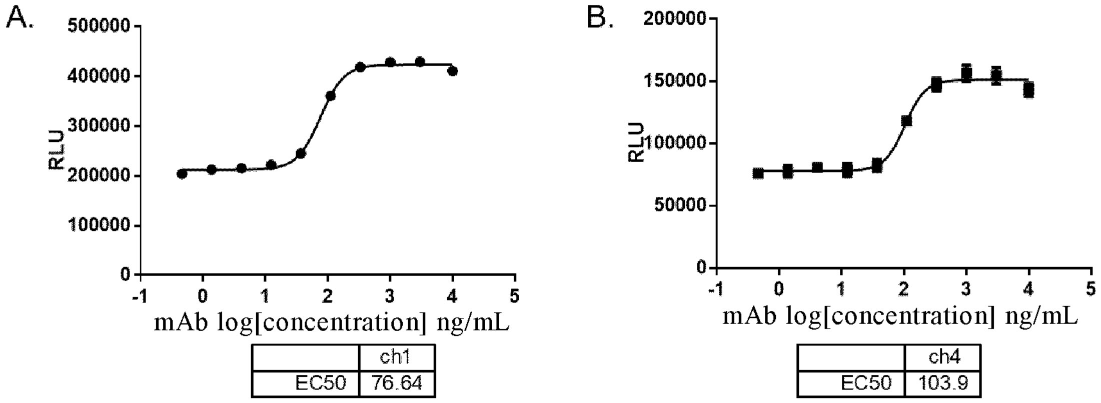


| | ch1 |
|---|---|
| EC50 | 76.64 |

| | ch4 |
|---|---|
| EC50 | 103.9 |

FIG. 3

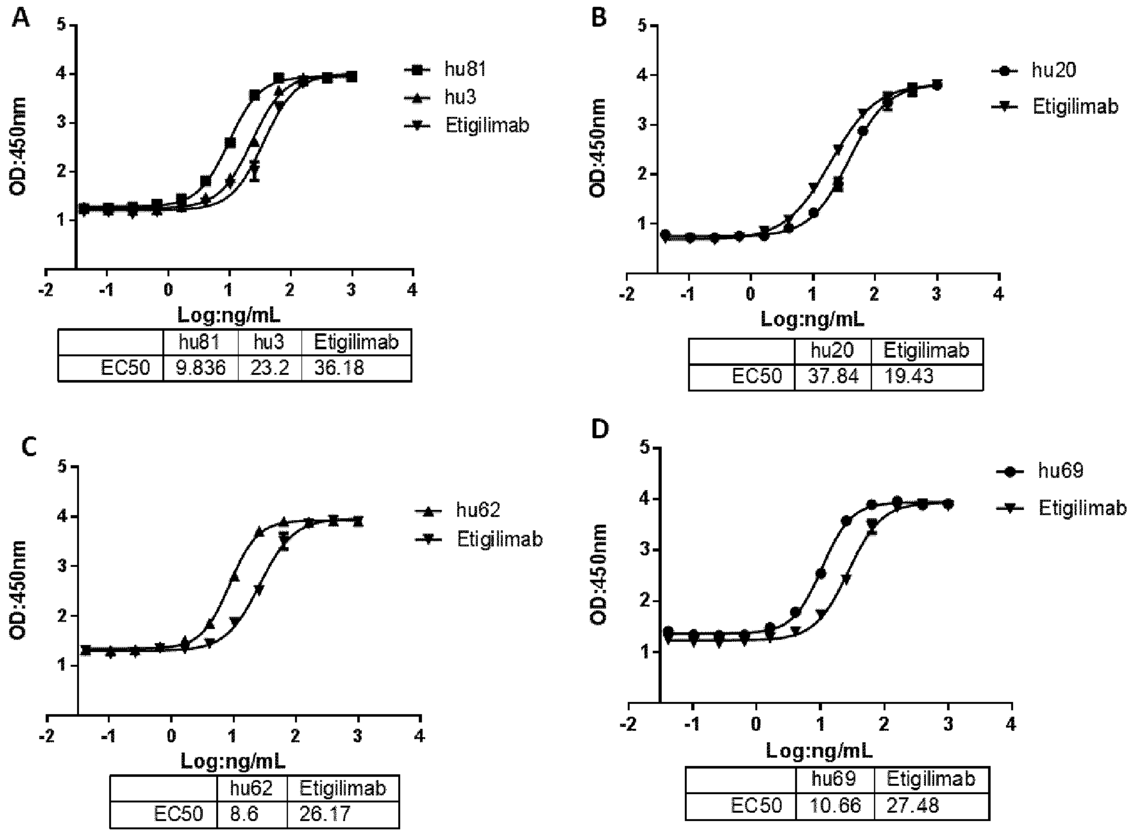


| | hu81 | hu3 | Etigilimab |
|---|---|---|---|
| EC50 | 9.836 | 23.2 | 36.18 |

| | hu20 | Etigilimab |
|---|---|---|
| EC50 | 37.84 | 19.43 |

| | hu62 | Etigilimab |
|---|---|---|
| EC50 | 8.6 | 26.17 |

| | hu69 | Etigilimab |
|---|---|---|
| EC50 | 10.66 | 27.48 |

FIG. 4

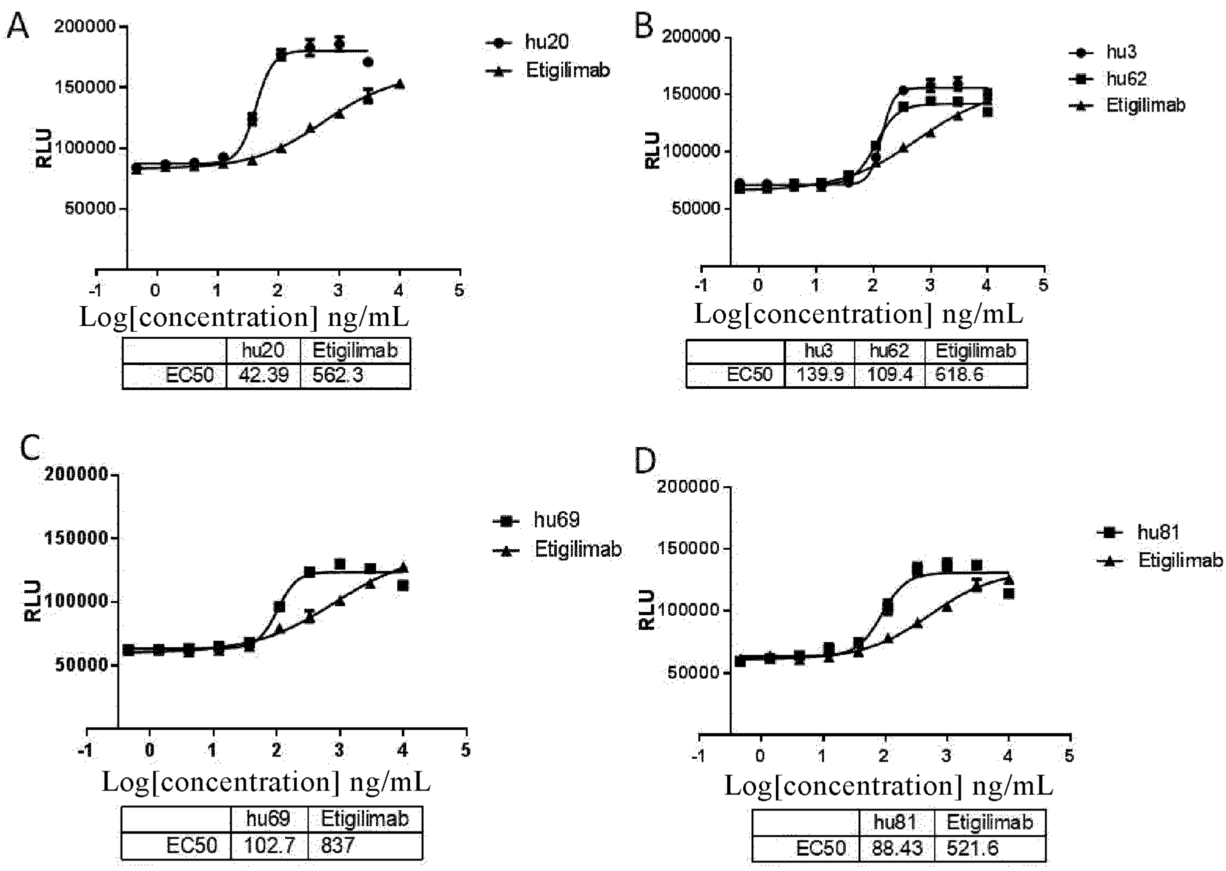
A
hu20
Etigilimab
RLU
Log[concentration] ng/mL
| hu20 | Etigilimab
EC50 | 42.39 | 562.3
B
hu3
hu62
Etigilimab
RLU
Log[concentration] ng/mL
| hu3 | hu62 | Etigilimab
EC50 | 139.9 | 109.4 | 618.6
C
hu69
Etigilimab
RLU
Log[concentration] ng/mL
| hu69 | Etigilimab
EC50 | 102.7 | 837
D
hu81
Etigilimab
RLU
Log[concentration] ng/mL
| hu81 | Etigilimab
EC50 | 88.43 | 521.6

FIG. 5

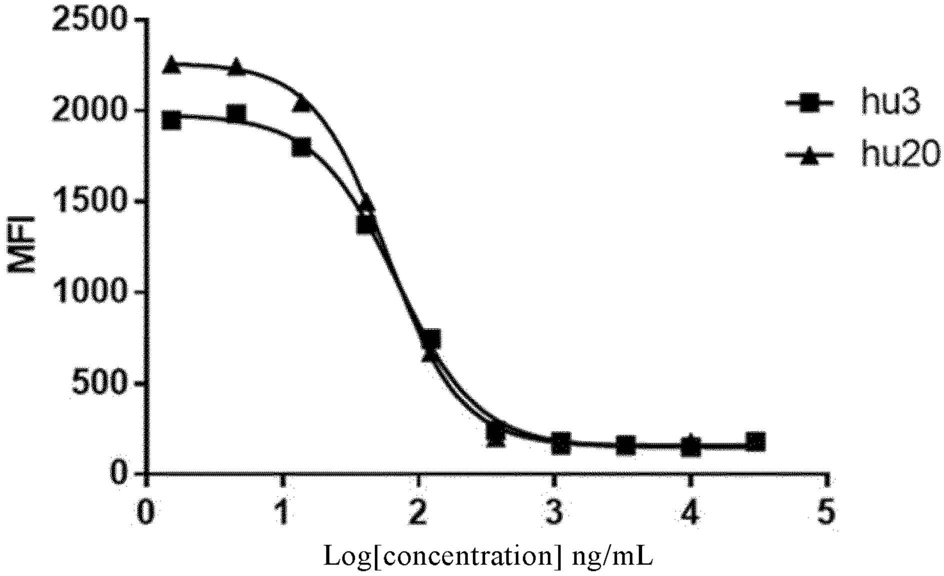


| | hu3 | hu20 |
|---|---|---|
| IC50 | 69.29 | 58.69 |

FIG. 6

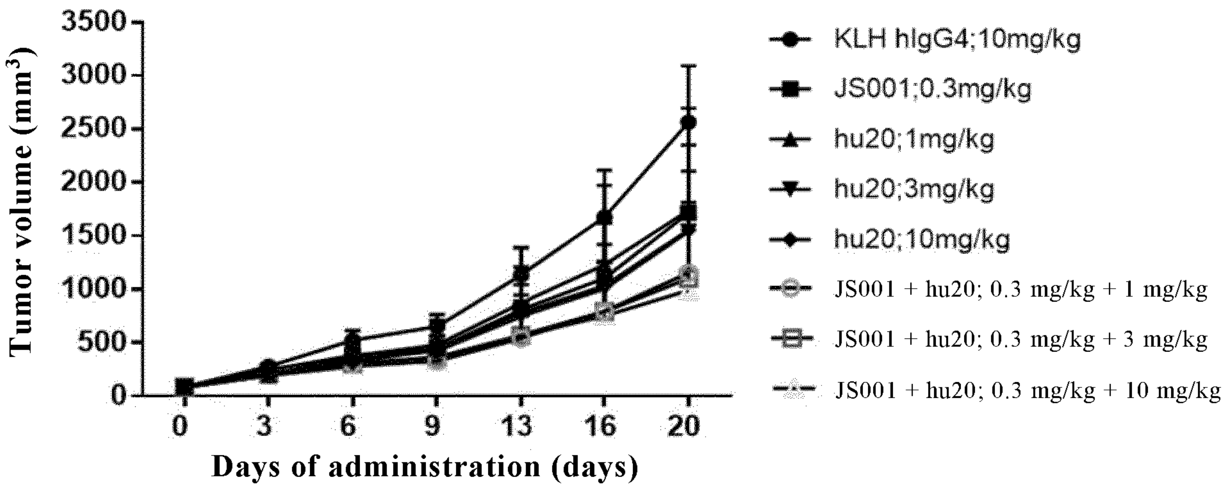


FIG. 7

## INTERNATIONAL SEARCH REPORT

International application No.
**PCT/CN2020/101883**

**A. CLASSIFICATION OF SUBJECT MATTER**

C07K 16/28(2006.01)i；C12N 15/13(2006.01)i；A61K 39/395(2006.01)i；A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, VEN, CNTXT, WOTXT, USTXT, EPTXT, JPTXT, CNKI, Web of Science, 百度学术, BAIDU XUESHU, Patentics, 中国生物序列检索系统, Chinese Biological Sequence Retrieval Database, Bio-Sequence Database of Chinese Patent, NCBI, EBI, STN: 申请人/发明人, applicant/inventor, 抗体, antibody, TIGIT, 含Ig和ITIM结构域的T细胞免疫受体, T cell immune receptor containing Ig and ITIM domains, 序列1-34, sequences 1-34

**C. DOCUMENTS CONSIDERED TO BE RELEVANT**

| Category* | Citation of document, with indication, where appropriate, of the relevant passages | Relevant to claim No. |
|---|---|---|
| A | CN 109384846 A (HEFEI RUIDA IMMUNE DRUGS RESEARCH INSTITUTE CO., LTD.) 26 February 2019 (2019-02-26)<br>entire document | 1-20 |
| A | CN 109071656 A (GENSUN BIOPHARMA INC.) 21 December 2018 (2018-12-21)<br>entire document | 1-20 |
| A | CN 108290936 A (MERCK SHARP & DOHME CORP.) 17 July 2018 (2018-07-17)<br>entire document | 1-20 |
| A | CN 108368176 A (POTENZA THERAPEUTICS INC.) 03 August 2018 (2018-08-03)<br>entire document | 1-20 |
| A | CN 107148430 A (MERCK SHARP & DOHME CORP.) 08 September 2017 (2017-09-08)<br>entire document | 1-20 |
| A | CN 108137691 A (YISSUM RESEARCH DEVELOPMENT COMPANY OF THE HEBREW UNIVERSITY OF JERUSALEM LTD. et al.) 08 June 2018 (2018-06-08)<br>entire document | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 September 2020** | **22 October 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2020/101883

C. DOCUMENTS CONSIDERED TO BE RELEVANT

| Category* | Citation of document, with indication, where appropriate, of the relevant passages | Relevant to claim No. |
|---|---|---|
| A | CN 107207594 A (BRISTOL-MYERS SQUIBB COMPANY) 26 September 2017 (2017-09-26)<br>entire document | 1-20 |
| A | WO 2018033798 A1 (COMPUGEN LTD.) 22 February 2018 (2018-02-22)<br>entire document | 1-20 |
| A | WO 2018102746 A1 (RIGEL PHARMACEUTICALS, INC.) 07 June 2018 (2018-06-07)<br>entire document | 1-20 |
| A | WO 2018204363 A1 (AGENUS INC.) 08 November 2018 (2018-11-08)<br>entire document | 1-20 |

Form PCT/ISA/210 (second sheet) (January 2015)

**INTERNATIONAL SEARCH REPORT**

International application No.
**PCT/CN2020/101883**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-17**
because they relate to subject matter not required to be searched by this Authority, namely:

[1] Claims 15-17 relate to a method for the treatment of cancer in a subject, and the subject matter of which does not warrant a search by the International Searching Authority according to the criteria set out in PCT Rule 39.1(iv). This international search is made on the basis of the use of the anti-TIGIT antibody or antigen-binding fragment thereof or the pharmaceutical composition in the preparation of drug for the treatment of cancer in subject.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/101883**

| Patent document cited in search report | | | Publication date (day/month/year) | Patent family member(s) | | | Publication date (day/month/year) |
|---|---|---|---|---|---|---|---|
| CN | 109384846 | A | 26 February 2019 | CN | 109384846 | B | 03 March 2020 |
| CN | 109071656 | A | 21 December 2018 | US | 2018185482 | A1 | 05 July 2018 |
| | | | | CN | 109988237 | A | 09 July 2019 |
| | | | | EP | 3565839 | A1 | 13 November 2019 |
| | | | | US | 2020164071 | A1 | 28 May 2020 |
| | | | | US | 10537637 | B2 | 21 January 2020 |
| | | | | WO | 2018128939 | A1 | 12 July 2018 |
| CN | 108290936 | A | 17 July 2018 | CA | 2994555 | A1 | 23 February 2017 |
| | | | | AU | 2016307845 | A1 | 08 March 2018 |
| | | | | EP | 3334757 | A4 | 03 April 2019 |
| | | | | US | 2018371083 | A1 | 27 December 2018 |
| | | | | EP | 3334757 | A2 | 20 June 2018 |
| | | | | JP | 2018527919 | A | 27 September 2018 |
| | | | | WO | 2017030823 | A3 | 30 March 2017 |
| | | | | WO | 2017030823 | A2 | 23 February 2017 |
| CN | 108368176 | A | 03 August 2018 | CO | 2018004743 | A2 | 30 November 2018 |
| | | | | WO | 2017059095 | A1 | 06 April 2017 |
| | | | | AU | 2016331052 | A1 | 26 April 2018 |
| | | | | PH | 12018500714 | A1 | 15 October 2018 |
| | | | | JP | 2018533371 | A | 15 November 2018 |
| | | | | AR | 109625 | A1 | 09 January 2019 |
| | | | | CA | 3000404 | A1 | 06 April 2017 |
| | | | | US | 9713641 | B2 | 25 July 2017 |
| | | | | US | 10507244 | B2 | 17 December 2019 |
| | | | | BR | 112018006531 | A2 | 11 December 2018 |
| | | | | MX | 2018003898 | A | 17 December 2018 |
| | | | | IL | 258394 | D0 | 31 May 2018 |
| | | | | US | 2020101158 | A1 | 02 April 2020 |
| | | | | SG | 10201912736U | A | 27 February 2020 |
| | | | | US | 2017340735 | A1 | 30 November 2017 |
| | | | | KR | 20180068990 | A | 22 June 2018 |
| | | | | EP | 3356413 | A1 | 08 August 2018 |
| | | | | HK | 1253235 | A1 | 14 June 2019 |
| | | | | TW | 201718636 | A | 01 June 2017 |
| | | | | US | 2017165366 | A1 | 15 June 2017 |
| CN | 107148430 | A | 08 September 2017 | AU | 2015305754 | B2 | 25 October 2018 |
| | | | | TN | 2017000024 | A1 | 04 July 2018 |
| | | | | CA | 2957722 | A1 | 25 February 2016 |
| | | | | CR | 20170060 | A | 18 April 2017 |
| | | | | US | 10618958 | B2 | 14 April 2020 |
| | | | | CL | 2019001926 | A1 | 29 November 2019 |
| | | | | CL | 2017000310 | A1 | 18 August 2017 |
| | | | | DO | P2017000046 | A | 31 August 2017 |
| | | | | AP | 201709765 | A0 | 28 February 2017 |
| | | | | US | 2017198042 | A1 | 13 July 2017 |
| | | | | US | 2016355589 | A1 | 08 December 2016 |
| | | | | AU | 2015305754 | A1 | 23 February 2017 |
| | | | | MD | 20170032 | A2 | 31 August 2017 |
| | | | | EA | 201790413 | A1 | 31 August 2017 |
| | | | | SG | 11201701161Q | A | 30 March 2017 |

Form PCT/ISA/210 (patent family annex) (January 2015)

**INTERNATIONAL SEARCH REPORT**
**Information on patent family members**

International application No.
**PCT/CN2020/101883**

| Patent document cited in search report | Publication date (day/month/year) | Patent family member(s) | Publication date (day/month/year) |
|---|---|---|---|
| | | IL 250583 D0 | 30 April 2017 |
| | | GT 201700033 A | 30 September 2019 |
| | | TW 201609813 A | 16 March 2016 |
| | | AP 201709765 D0 | 28 February 2017 |
| | | PH 12017500296 A1 | 28 June 2017 |
| | | MX 2017002229 A | 09 May 2017 |
| | | AU 2019200426 A1 | 07 February 2019 |
| | | NI 201700019 A | 08 March 2017 |
| | | KR 20170041272 A | 14 April 2017 |
| | | PE 20170289 A1 | 05 April 2017 |
| | | WO 2016028656 A1 | 25 February 2016 |
| | | JP 2017532007 A | 02 November 2017 |
| | | AP 2017009765 A0 | 28 February 2017 |
| | | EP 3183267 A1 | 28 June 2017 |
| | | BR 112017003108 A2 | 05 December 2017 |
| | | US 2018066055 A1 | 08 March 2018 |
| | | KR 20190133078 A | 29 November 2019 |
| | | KR 102050082 B1 | 29 November 2019 |
| CN 108137691 A | 08 June 2018 | JP 2018532383 A | 08 November 2018 |
| | | US 2018185480 A1 | 05 July 2018 |
| | | EP 3344658 A1 | 11 July 2018 |
| | | WO 2017037707 A1 | 09 March 2017 |
| | | CA 2997130 A1 | 09 March 2017 |
| CN 107207594 A | 26 September 2017 | MX 2017007744 A | 05 September 2017 |
| | | EP 3237448 A1 | 01 November 2017 |
| | | US 2016176963 A1 | 23 June 2016 |
| | | TN 2017000267 A1 | 19 October 2018 |
| | | CL 2017001660 A1 | 23 March 2018 |
| | | PH 12017501166 A1 | 11 December 2017 |
| | | JP 2020062026 A | 23 April 2020 |
| | | US 2019112375 A1 | 18 April 2019 |
| | | JP 6633032 B2 | 22 January 2020 |
| | | JP 2018035138 A | 08 March 2018 |
| | | UY 36471 A | 30 June 2016 |
| | | AR 103268 A1 | 26 April 2017 |
| | | KR 20170099966 A | 01 September 2017 |
| | | WO 2016106302 A1 | 30 June 2016 |
| | | EA 201791171 A1 | 30 November 2017 |
| | | IL 253013 D0 | 31 August 2017 |
| | | ZA 201704981 B | 28 August 2019 |
| | | CN 110256558 A | 20 September 2019 |
| | | PE 20171244 A1 | 24 August 2017 |
| | | JP 6180663 B2 | 16 August 2017 |
| | | CO 2017006989 A2 | 05 January 2018 |
| | | AU 2015369683 A1 | 10 August 2017 |
| | | TW 201629102 A | 16 August 2016 |
| | | WO 2016106302 A9 | 22 June 2017 |
| | | JP 2017520512 A | 27 July 2017 |
| | | SG 11201705063V A | 28 July 2017 |
| | | CA 2971732 A1 | 30 June 2016 |

Form PCT/ISA/210 (patent family annex) (January 2015)

**INTERNATIONAL SEARCH REPORT**
**Information on patent family members**

International application No.
**PCT/CN2020/101883**

| Patent document cited in search report | Publication date (day/month/year) | Patent family member(s) | Publication date (day/month/year) |
|---|---|---|---|
| | | BR 112017013385 A2 | 06 February 2018 |
| | | CN 107207594 B | 07 May 2019 |
| | | US 10189902 B2 | 29 January 2019 |
| WO 2018033798 A1 | 22 February 2018 | SG 11201901077 R A | 28 March 2019 |
| | | ES 2774320 T3 | 20 July 2020 |
| | | HR P20200189 T1 | 01 May 2020 |
| | | CA 3032331 A1 | 22 February 2018 |
| | | SI 3347379 T1 | 30 April 2020 |
| | | EP 3347379 B1 | 06 November 2019 |
| | | DK 3347379 T3 | 17 February 2020 |
| | | US 10124061 B2 | 13 November 2018 |
| | | CL 2019000424 A1 | 05 July 2019 |
| | | AU 2017313405 A1 | 28 February 2019 |
| | | CN 110088132 A | 02 August 2019 |
| | | DK 3347379 T5 | 15 June 2020 |
| | | US 2018280506 A1 | 04 October 2018 |
| | | PL 3347379 T3 | 18 May 2020 |
| | | PT 3347379 T | 18 February 2020 |
| | | EP 3347379 B9 | 25 March 2020 |
| | | ES 2774320 T9 | 21 July 2020 |
| | | KR 20190039421 A | 11 April 2019 |
| | | MX 2019001878 A | 01 July 2019 |
| | | US 2018305456 A1 | 25 October 2018 |
| | | BR 112019003129 A2 | 09 July 2019 |
| | | JP 2019533984 A | 28 November 2019 |
| | | EP 3617232 A1 | 04 March 2020 |
| | | CO 2019001980 A2 | 08 March 2019 |
| | | EP 3347379 A1 | 18 July 2018 |
| | | LT 3347379 T | 11 May 2020 |
| | | US 2018169238 A1 | 21 June 2018 |
| | | US 10213505 B2 | 26 February 2019 |
| WO 2018102746 A1 | 07 June 2018 | US 2018155422 A1 | 07 June 2018 |
| WO 2018204363 A1 | 08 November 2018 | US 2018355040 A1 | 13 December 2018 |
| | | AU 2018263857 A1 | 21 November 2019 |
| | | EP 3618863 A1 | 11 March 2020 |
| | | CA 3062061 A1 | 08 November 2018 |
| | | TW 201843178 A | 16 December 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004024068 A
- WO 2009126688 A
- WO 2015009856 A
- WO 2016028656 A
- WO 2014206107 A
- WO 2018153320 A
- US 8278036 B, Kariko
- US 4816567 A, Cabilly
- US 5225539 A, Winter
- US 5530101 A
- US 5585089 A
- US 5693762 A
- US 6180370 B, Queen
- CN 201310258289
- CN 2013102582892


### Non-patent literature cited in the description


- **STANIETSKY et al.** *PNAS,* 2009, vol. 106, 17858-17863
- **LIU et al.** *Cell death and differentiation,* 2013, vol. 20, 456-464
- **STANIETSKY et al.** *European journal of immunology,* 2013, vol. 43, 2138-2150
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **KOHLER et al.** *Nature,* 1975, vol. 256, 495-497
- **LIU et al.** *Proc. Natl. Acad. Sci. USA,* 1987, vol. 84, 3439-43
- **JONES et al.** *Nature,* 1986, vol. 321, 522
- **VERHOEYEN et al.** *Science,* 1988, vol. 239, 1534
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** *Sequences of Proteins of Immunological Interest,* 1987
- *Nucleic Acids Research,* 1999, vol. 27, 209-212
- Remington's Pharmaceutical Sciences. 1980

SEQUENCE LISTING

<110> Shanghai Junshi Biosciences Co., Ltd.
Suzhou Junmeng Biosciences Co., Ltd.

<120> ANTI-TIGIT ANTIBODIES AND APPLICATION THEREOF

<130> 204340 1PCWO

<150> CN 201910634309.9
<151> 2019-07-15

<160> 34

<170> SIPOSequenceListing 1.0

<210> 1
<211> 8
<212> PRT
<213> Mus musculus

<400> 1
```
Gly Tyr Ala Phe Thr Glu Tyr Thr
1               5
```

<210> 2
<211> 8
<212> PRT
<213> Mus musculus

<400> 2
```
Ile Asn Pro Asn Thr Gly Gly Thr
1               5
```

<210> 3
<211> 12
<212> PRT
<213> Mus musculus

<400> 3
```
Ala Lys Leu Leu Arg Leu Met Tyr Tyr Phe Asp Tyr
1               5                   10
```

<210> 4
<211> 119
<212> PRT
<213> Mus musculus

<400> 4
```
Glu Val Gln Leu Gln Gln Ser Gly Pro Glu Leu Val Lys Pro Gly Ala
1               5                   10                  15
Ser Val Lys Ile Ser Cys Lys Thr Ser Gly Tyr Ala Phe Thr Glu Tyr
            20                  25                  30
Thr Met His Trp Val Lys Gln Ser His Gly Lys Ser Leu Glu Trp Ile
        35                  40                  45
Gly Gly Ile Asn Pro Asn Thr Gly Gly Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Phe Arg Ser Leu Thr Ser Glu Asp Ser Ala Val Tyr Tyr Cys
                85                  90                  95
Ala Lys Leu Leu Arg Leu Met Tyr Tyr Phe Asp Tyr Trp Gly Gln Gly
            100                 105                 110
```

```
Thr Thr Leu Thr Val Ser Ser
        115

<210>  5
<211>  357
<212>  DNA
<213>  Mus musculus

<400>  5
gaggtccagc tgcaacagtc tggacctgag ctggtgaagc ctggggcttc agtgaagata         60
tcctgcaaga cttctggata cgcattcact gaatacacca tgcactgggt gaaacagagc        120
catggaaaga gccttgagtg gattggaggt attaatccta acactggtgg tactacctac        180
aaccagaagt tcaagggcaa ggccacattg actgtagaca agtcctccag cacagcctac        240
atggagttcc gcagcctgac atctgaggat tctgcagtct attactgtgc aaaattacta        300
cggctcatgt actactttga ctactggggc caaggcacca ctctcacagt ctcctca           357

<210>  6
<211>  6
<212>  PRT
<213>  Mus musculus

<400>  6
Gln Asp Val Arg Thr Ala
1               5

<210>  7
<211>  3
<212>  PRT
<213>  Mus musculus

<400>  7
Ser Ala Ser
1

<210>  8
<211>  9
<212>  PRT
<213>  Mus musculus

<400>  8
His Gln His Tyr Ile Thr Pro Trp Thr
1               5

<210>  9
<211>  107
<212>  PRT
<213>  Mus musculus

<400>  9
Asp Ile Val Met Thr Gln Ser His Lys Phe Met Ser Thr Ser Val Gly
1               5                   10                  15
Asp Arg Val Ser Ile Thr Cys Lys Ala Ser Gln Asp Val Arg Thr Ala
            20                  25                  30
Val Ala Trp Tyr Gln Gln Lys Pro Gly Gln Ser Pro Lys Leu Leu Ile
        35                  40                  45
Tyr Ser Ala Ser Tyr Arg Tyr Thr Gly Val Pro Asp Arg Phe Thr Gly
    50                  55                  60
Ser Gly Ser Gly Thr Asp Phe Thr Phe Thr Ile Ser Ser Val Gln Ala
65                  70                  75                  80
Glu Asp Leu Ala Leu Tyr Tyr Cys His Gln His Tyr Ile Thr Pro Trp
                85                  90                  95
Thr Phe Gly Gly Gly Thr Lys Leu Glu Ile Lys
            100                 105
```

<210> 10
<211> 321
<212> DNA
<213> Mus musculus

<400> 10
gacattgtga tgacccagtc tcacaaattc atgtccacat cagtaggaga cagggtcagc 60
atcacctgca aggccagtca ggatgtgagg actgctgtag cctggtatca acagaaacca 120
ggacaatctc ctaaactact gatttactcg gcatcctacc ggtacactgg agtccctgat 180
cgcttcactg gcagtggatc tgggacggat ttcactttca ccatcagcag tgtgcaggct 240
gaagacctgg cactttatta ctgtcatcaa cattatatta ctccgtggac gttcggtgga 300
ggcaccaagc tggaaatcaa a 321

<210> 11
<211> 8
<212> PRT
<213> Mus musculus

<400> 11
Gly Tyr Thr Phe Thr Asn Tyr Trp
1 5

<210> 12
<211> 8
<212> PRT
<213> Mus musculus

<400> 12
Ile Tyr Pro Gly Gly His Tyr Thr
1 5

<210> 13
<211> 11
<212> PRT
<213> Mus musculus

<400> 13
Thr Arg Tyr Tyr Gly Pro Tyr Ala Met Asp Tyr
1 5 10

<210> 14
<211> 118
<212> PRT
<213> Mus musculus

<400> 14
Gln Val Gln Leu Gln Gln Ser Gly Asp Glu Leu Val Arg Pro Gly Thr
1 5 10 15
Ser Val Lys Val Ser Cys Gln Ala Ala Gly Tyr Thr Phe Thr Asn Tyr
20 25 30
Trp Ile Leu Trp Ile Lys Gln Arg Pro Arg His Gly Leu Glu Trp Ile
35 40 45
Gly Asp Ile Tyr Pro Gly Gly His Tyr Thr Asn Tyr Asn Glu Lys Phe
50 55 60
Lys Gly Lys Ala Thr Leu Thr Ala Asp Thr Ser Ser Ser Thr Ala Tyr
65 70 75 80
Met Gln Leu Asn Ser Leu Thr Ser Glu Asp Ser Ala Ile Tyr Tyr Cys
85 90 95
Thr Arg Tyr Tyr Gly Pro Tyr Ala Met Asp Tyr Trp Gly Gln Gly Thr
100 105 110
Ser Val Thr Val Ser Ser
115

<210> 15
<211> 354
<212> DNA
<213> Mus musculus

<400> 15
caggtccagc tgcagcagtc tggagatgaa ctggtaaggc ctgggacttc agtgaaggtg 60
tcctgccagg ctgctggata caccttcact aactactgga tactttggat aaagcagagg 120
cctcgacatg gccttgagtg gattggagat atttaccctg gaggtcatta tactaattac 180
aatgagaaat tcaagggcaa ggccacactg actgcagaca catcctccag tacagcctac 240
atgcaactca acagcctgac atctgaggac tctgccatct attactgtac aagatactat 300
ggtccctatg ctatggacta ctggggtcaa ggaacctcag tcaccgtctc ctca 354

<210> 16
<211> 12
<212> PRT
<213> Mus musculus

<400> 16
Gln Ser Leu Leu Tyr Asn Ser Asn Gln Lys Asn Tyr
1 5 10

<210> 17
<211> 3
<212> PRT
<213> Mus musculus

<400> 17
Trp Ala Ser
1

<210> 18
<211> 9
<212> PRT
<213> Mus musculus

<400> 18
Gln Gln Tyr Tyr Ser Tyr Pro Phe Thr
1 5

<210> 19
<211> 113
<212> PRT
<213> Mus musculus

<400> 19
Asp Ile Val Met Ser Gln Ser Pro Ser Ser Leu Pro Val Ser Val Gly
1 5 10 15
Glu Lys Val Thr Met Ser Cys Lys Ser Ser Gln Ser Leu Leu Tyr Asn
20 25 30
Ser Asn Gln Lys Asn Tyr Leu Ala Trp Tyr Gln Gln Ile Pro Gly Gln
35 40 45
Ser Pro Lys Leu Leu Ile Tyr Trp Ala Ser Thr Gly Glu Ser Gly Val
50 55 60
Pro Asp Arg Phe Thr Gly Ser Gly Ser Gly Thr Asp Phe Thr Leu Thr
65 70 75 80
Ile Ser Ser Val Lys Ala Glu Asp Leu Ala Val Tyr Tyr Cys Gln Gln
85 90 95
Tyr Tyr Ser Tyr Pro Phe Thr Phe Gly Ser Gly Thr Lys Leu Glu Ile
100 105 110
Lys

<210> 20
<211> 339
<212> DNA
<213> Mus musculus

<400> 20
gacattgtga tgtcacagtc tccatcctcc ctacctgtgt cagttggaga gaaggttact 60
atgagctgca agtccagtca gagcctttta tataatagta atcaaaagaa ctacttggcc 120
tggtaccagc agataccagg gcagtctcct aaactgctga tttactgggc atccactggg 180
gaatctgggg tccctgatcg cttcacaggc agtggatctg ggacagattt cactctcacc 240
atcagcagtg tgaaggctga agacctggca gtttattact gtcagcaata ttatagctat 300
ccattcacgt tcggctcggg gacaaagttg gaaataaaa 339

<210> 21
<211> 119
<212> PRT
<213> Artificial sequence

<220>
<223> Amino acid sequence of heavy chain variable region

<400> 21
Gln Val Gln Leu Val Gln Ser Gly Ala Glu Val Lys Lys Pro Gly Ala
1 5 10 15
Ser Val Lys Val Ser Cys Lys Thr Ser Gly Tyr Ala Phe Thr Glu Tyr
20 25 30
Thr Met His Trp Val Arg Gln Ala Pro Gly Gln Gly Leu Glu Trp Met
35 40 45
Gly Gly Ile Asn Pro Asn Thr Gly Gly Thr Thr Tyr Asn Gln Lys Phe
50 55 60
Gln Gly Arg Val Thr Leu Thr Val Asp Lys Ser Ser Ser Thr Ala Tyr
65 70 75 80
Met Glu Leu Ser Arg Leu Arg Ser Asp Asp Thr Val Val Tyr Tyr Cys
85 90 95
Ala Lys Leu Leu Arg Leu Met Tyr Tyr Phe Asp Tyr Trp Gly Gln Gly
100 105 110
Thr Leu Val Thr Val Ser Ser
115

<210> 22
<211> 107
<212> PRT
<213> Artificial sequence

<220>
<223> Amino acid sequence of light chain variable region

<400> 22
Asp Ile Val Met Thr Gln Ser Pro Asp Ser Leu Ala Val Ser Leu Gly
1 5 10 15
Glu Arg Ala Thr Ile Asn Cys Lys Ala Ser Gln Asp Val Arg Thr Ala
20 25 30
Val Ala Trp Tyr Gln Gln Lys Pro Gly Gln Pro Pro Lys Leu Leu Ile
35 40 45
Tyr Ser Ala Ser Tyr Arg Tyr Thr Gly Val Pro Asp Arg Phe Ser Gly
50 55 60
Ser Gly Ser Gly Thr Asp Phe Thr Leu Thr Ile Ser Ser Leu Gln Ala
65 70 75 80
Glu Asp Val Ala Val Tyr Tyr Cys His Gln His Tyr Ile Thr Pro Trp
85 90 95
Thr Phe Gly Gly Gly Thr Lys Val Glu Ile Lys
100 105

<210> 23
<211> 119
<212> PRT
<213> Artificial sequence

<220>
<223> Amino acid sequence of heavy chain variable region

<400> 23
```
Gln Val Gln Leu Val Gln Ser Gly Ala Glu Val Lys Lys Pro Gly Ala
1               5                   10                  15
Ser Val Lys Val Ser Cys Lys Thr Ser Gly Tyr Ala Phe Thr Glu Tyr
            20                  25                  30
Thr Met His Trp Val Arg Gln Ala Pro Gly Lys Gly Leu Glu Trp Met
        35                  40                  45
Gly Gly Ile Asn Pro Asn Thr Gly Gly Thr Thr Tyr Asn Gln Lys Phe
    50                  55                  60
Gln Gly Arg Val Thr Leu Thr Val Asp Lys Ser Ser Ser Thr Ala Tyr
65                  70                  75                  80
Met Glu Leu Ser Ser Leu Arg Ser Glu Asp Thr Val Val Tyr Tyr Cys
                85                  90                  95
Ala Lys Leu Leu Arg Leu Met Tyr Tyr Phe Asp Tyr Trp Gly Gln Gly
            100                 105                 110
Thr Leu Val Thr Val Ser Ser
        115
```

<210> 24
<211> 107
<212> PRT
<213> Artificial sequence

<220>
<223> Amino acid sequence of light chain variable region

<400> 24
```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Val Arg Thr Ala
            20                  25                  30
Val Ala Trp Tyr Gln Gln Lys Pro Gly Lys Ala Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys His Gln His Tyr Ile Thr Pro Trp
                85                  90                  95
Thr Phe Gly Gly Gly Thr Lys Val Glu Ile Lys
            100                 105
```

<210> 25
<211> 118
<212> PRT
<213> Artificial sequence

<220>
<223> Amino acid sequence of heavy chain variable region

<400> 25
```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
```

```
                20                  25                  30
Trp Ile Leu Trp Ile Arg Gln Ala Pro Gly Gln Gly Leu Glu Trp Met
            35                  40                  45
Gly Asp Ile Tyr Pro Gly Gly His Tyr Thr Asn Tyr Asn Glu Lys Phe
    50                  55                  60
Gln Gly Arg Val Thr Leu Thr Ala Asp Thr Ser Ser Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Tyr Tyr Gly Pro Tyr Ala Met Asp Tyr Trp Gly Gln Gly Thr
            100                 105                 110
Leu Val Thr Val Ser Ser
        115
```

<210> 26
<211> 113
<212> PRT
<213> Artificial sequence

<220>
<223> Amino acid sequence of light chain variable region

<400> 26

```
Asp Ile Gln Met Thr Gln Ser Pro Ser Ser Leu Ser Ala Ser Val Gly
1               5                   10                  15
Asp Arg Val Thr Ile Thr Cys Arg Ser Ser Gln Ser Leu Leu Tyr Asn
            20                  25                  30
Ser Asn Gln Lys Asn Tyr Leu Ala Trp Tyr Gln Gln Lys Pro Gly Lys
        35                  40                  45
Ala Pro Lys Leu Leu Ile Tyr Trp Ala Ser Thr Gly Glu Ser Gly Val
    50                  55                  60
Pro Ser Arg Phe Ser Gly Ser Gly Ser Gly Thr Asp Phe Thr Leu Thr
65                  70                  75                  80
Ile Ser Ser Leu Gln Pro Glu Asp Phe Ala Thr Tyr Tyr Cys Gln Gln
                85                  90                  95
Tyr Tyr Ser Tyr Pro Phe Thr Phe Gly Gln Gly Thr Lys Val Glu Ile
            100                 105                 110
Lys
```

<210> 27
<211> 118
<212> PRT
<213> Artificial sequence

<220>
<223> Amino acid sequence of heavy chain variable region

<400> 27

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Trp Ile Leu Trp Ile Arg Gln Ala Pro Gly Gln Gly Leu Glu Trp Met
        35                  40                  45
Gly Asp Ile Tyr Pro Gly Gly His Tyr Thr Asn Tyr Asn Glu Lys Phe
    50                  55                  60
Gln Gly Arg Val Thr Leu Thr Ala Asp Thr Ser Ser Ser Thr Ala Tyr
65                  70                  75                  80
Met Glu Leu Ser Arg Leu Arg Ser Asp Asp Thr Ala Val Tyr Tyr Cys
                85                  90                  95
Thr Arg Tyr Tyr Gly Pro Tyr Ala Met Asp Tyr Trp Gly Gln Gly Thr
            100                 105                 110
```

```
Leu Val Thr Val Ser Ser
        115

<210>  28
<211>  113
<212>  PRT
<213>  Artificial sequence

<220>
<223>  Amino acid sequence of light chain variable region

<400>  28
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu Tyr Asn
            20                  25                  30
Ser Asn Gln Lys Asn Tyr Leu Ala Trp Tyr Leu Gln Lys Pro Gly Gln
        35                  40                  45
Ser Pro Gln Leu Leu Ile Tyr Trp Ala Ser Thr Gly Glu Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Gln Gln
                85                  90                  95
Tyr Tyr Ser Tyr Pro Phe Thr Phe Gly Gln Gly Thr Lys Leu Glu Ile
            100                 105                 110
Lys


<210>  29
<211>  118
<212>  PRT
<213>  Artificial sequence

<220>
<223>  Amino acid sequence of heavy chain variable region

<400>  29
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Trp Ile Leu Trp Ile Arg Gln Ala Pro Gly Gln Gly Leu Glu Trp Met
        35                  40                  45
Gly Asp Ile Tyr Pro Gly Gly His Tyr Thr Asn Tyr Asn Glu Lys Leu
    50                  55                  60
Gln Gly Arg Val Thr Leu Thr Ala Asp Thr Ser Ser Ser Thr Ala Tyr
65                  70                  75                  80
Met Glu Leu Arg Ser Leu Arg Ser Asp Asp Thr Ala Val Tyr Tyr Cys
                85                  90                  95
Thr Arg Tyr Tyr Gly Pro Tyr Ala Met Asp Tyr Trp Gly Gln Gly Thr
            100                 105                 110
Leu Val Thr Val Ser Ser
        115

<210>  30
<211>  113
<212>  PRT
<213>  Artificial sequence

<220>
<223>  Amino acid sequence of light chain variable region
```

<400> 30
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu Tyr Asn
20 25 30
Ser Asn Gln Lys Asn Tyr Leu Ala Trp Tyr Leu Gln Lys Pro Gly Gln
35 40 45
Ser Pro Gln Leu Leu Ile Tyr Trp Ala Ser Thr Gly Glu Ser Gly Val
50 55 60
Pro Asp Arg Phe Ser Gly Ser Gly Ser Gly Thr Asp Phe Thr Leu Lys
65 70 75 80
Ile Ser Arg Val Glu Ala Glu Asp Val Gly Val Tyr Tyr Cys Gln Gln
85 90 95
Tyr Tyr Ser Tyr Pro Phe Thr Phe Gly Gln Gly Thr Lys Val Glu Ile
100 105 110
Lys

<210> 31
<211> 446
<212> PRT
<213> Artificial sequence

<220>
<223> Amino acid sequence of heavy chain

<400> 31
Gln Val Gln Leu Val Gln Ser Gly Ala Glu Val Lys Lys Pro Gly Ala
1 5 10 15
Ser Val Lys Val Ser Cys Lys Thr Ser Gly Tyr Ala Phe Thr Glu Tyr
20 25 30
Thr Met His Trp Val Arg Gln Ala Pro Gly Gln Gly Leu Glu Trp Met
35 40 45
Gly Gly Ile Asn Pro Asn Thr Gly Gly Thr Thr Tyr Asn Gln Lys Phe
50 55 60
Gln Gly Arg Val Thr Leu Thr Val Asp Lys Ser Ser Ser Thr Ala Tyr
65 70 75 80
Met Glu Leu Ser Arg Leu Arg Ser Asp Asp Thr Val Val Tyr Tyr Cys
85 90 95
Ala Lys Leu Leu Arg Leu Met Tyr Tyr Phe Asp Tyr Trp Gly Gln Gly
100 105 110
Thr Leu Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285

```
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Lys
        435                 440                 445

<210>  32
<211>  214
<212>  PRT
<213>  Artificial sequence

<220>
<223>  Amino acid sequence of light chain

<400>  32
Asp Ile Val Met Thr Gln Ser Pro Asp Ser Leu Ala Val Ser Leu Gly
1               5                   10                  15
Glu Arg Ala Thr Ile Asn Cys Lys Ala Ser Gln Asp Val Arg Thr Ala
            20                  25                  30
Val Ala Trp Tyr Gln Gln Lys Pro Gly Gln Pro Pro Lys Leu Leu Ile
        35                  40                  45
Tyr Ser Ala Ser Tyr Arg Tyr Thr Gly Val Pro Asp Arg Phe Ser Gly
    50                  55                  60
Ser Gly Ser Gly Thr Asp Phe Thr Leu Thr Ile Ser Ser Leu Gln Ala
65                  70                  75                  80
Glu Asp Val Ala Val Tyr Tyr Cys His Gln His Tyr Ile Thr Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  33
<211>  446
<212>  PRT
<213>  Artificial sequence
```

<220>
<223> Amino acid sequence of heavy chain

<400> 33

```
Gln Val Gln Leu Val Gln Ser Gly Ala Glu Val Lys Lys Pro Gly Ala
1               5                   10                  15
Ser Val Lys Val Ser Cys Lys Thr Ser Gly Tyr Ala Phe Thr Glu Tyr
            20                  25                  30
Thr Met His Trp Val Arg Gln Ala Pro Gly Lys Gly Leu Glu Trp Met
        35                  40                  45
Gly Gly Ile Asn Pro Asn Thr Gly Gly Thr Thr Tyr Asn Gln Lys Phe
    50                  55                  60
Gln Gly Arg Val Thr Leu Thr Val Asp Lys Ser Ser Ser Thr Ala Tyr
65                  70                  75                  80
Met Glu Leu Ser Ser Leu Arg Ser Glu Asp Thr Val Val Tyr Tyr Cys
                85                  90                  95
Ala Lys Leu Leu Arg Leu Met Tyr Tyr Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Lys
        435                 440                 445
```

<210> 34

```
<211>  214
<212>  PRT
<213>  Artificial sequence

<220>
<223>  Amino acid sequence of light chain

<400>  34
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Val Arg Thr Ala
            20                  25                  30
Val Ala Trp Tyr Gln Gln Lys Pro Gly Lys Ala Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys His Gln His Tyr Ile Thr Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```